# EUROPEAN PATENT APPLICATION

(11) **EP 1 580 189 A1**
(43) Date of publication of application: **28.09.2005**
(21) Application number: 03768254.9
(22) Date of filing: 25.12.2003
(51) Int. Cl.: C07D 231/12, C07D 231/14, C07D 231/16, C07D 231/18

(54) **4-AMINO-5-METHYLPYRAZOLE DERIVATIVES AND PROCESS FOR PRODUCTION THEREOF**

(30) Priority: 27.12.2002 JP 2002381360
(71) Applicant: Sankyo Agro Company, Limited, Tokyo 113-0033 (JP)
(72) Inventor: KAJINO, Hisaki, c/o SANKYO CO., LTD., Hiratsuka-shi, Kanagawa 254-0014 (JP); MORIMOTO, Munetsugu, c/o SANKYO AGRO COMPANY, LTD., Yasu-gun, Shiga 520-2342 (JP)
(74) Representative: Bohmann, Armin K.
(86) International application number: PCT/JP2003/016769
(87) International publication number: WO 2004/060877

(57) **Abstract**

The present invention relates to 4-amino-5-methylpyrazole derivatives represented by the following formula (I): 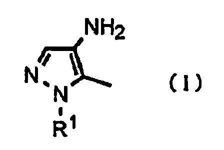 (wherein R¹ represents an isobutyl group, a cyclobutylmethyl group, a neopentyl group or the like) or a salt thereof, which are novel compounds being useful as synthetic intermediates of pharmaceuticals, agricultural chemicals or the like.

## Description

### [Technical field]

The present invention relates to 4-amino-5-methylpyrazole derivatives being useful as synthetic intermediates of pharmaceuticals, agricultural chemicals or the like, and preparation process thereof.

### [Background technique]

4-Acylaminopyrazole derivatives are compounds exhibiting excellent control effects against the injury of disease such as tomato late blight, grape downy mildew and rice seedling blight (see Patent reference 1: Japanese Unexamined Patent Publication No. 2002-138082).

The 4-acylaminopyrazole derivatives are synthesized from known compounds through various intermediates, but 4-amino-5-methylpyrazoles having a substituent at the 1-position of the pyrazole ring (hereinafter referred to "4-amino-5-methylpyrazoles") are also one of such intermediates.

There are not so many examples of report regarding the 4-amino-5-methylpyrazoles and compounds in which the substituent at the 1-position is a methyl group (4-amino-1,5-dimethylpyrazole, J. Chem. Soc., Perkin Trans., Vol. 24, 3721 (1999)) and compounds in which the substituent at the 1-position is a phenyl group (4-amino-5-methyl-1-phenylpyrazole, J. Chem. Soc., 3259 (1958)) are reported. In Patent Reference 1, it is described that the 4-amino-5-methylpyrazole having an isobutyl substituent at the 1-position (4-methyl-1-isobutyl-5-methyl-1H-pyrazole) is used, but it is not used as a single component but as a mixture with the 3-methyl derivative.

Thus one reason that the report regarding the 4-amino-5-methylpyrazoles is less, includes that an appropriate synthetic method is not established. As the synthetic method of the 4-amino-5-methylpyrazoles, (1) a lithiation method, (2) an acetoacetic acid ester method, (3) an acetylacetone method or the like are considered. In the following, these methods are described.

### (1) Lithiation method

The lithiation method is a method in which the desired 4-amino-5-methylpyrazoles represented by the formula (Ia) are obtained by alkylating 1H-pyrazole to obtain compounds represented by the formula (1-1) (Step A), then lithiating selectively the 5-position of the compounds (1-1) and introducing a methyl group to the 5-position using a methylating agent to obtain compounds represented by the formula (1-2) (Step B), and finally nitrating selectively the 4-position of the compounds (1-2) and reducing them (Step C). However, regarding any of each Steps, efficient methods have not been known in the reaction yields and operationality.

### Step A, Step B and Step C:

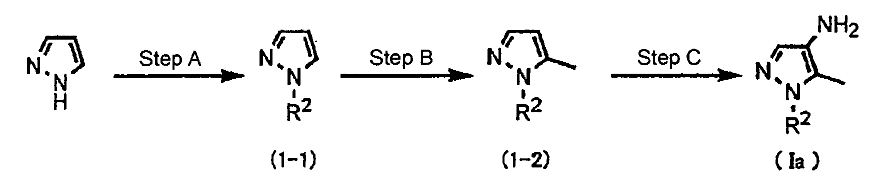

For example, in Step A, under usual conditions where a method to prepare the compounds represented by the general formula (1-1) is carried out, i.e., employing a strong base such as sodium hydride and a halogenated alkyl in a high polar solvent such as dimethylformamide, the desired compounds could hardly be obtained. Further, in Synthetic Commun., vol. 20, 2849 (1990), the method to react 1H-pyrazole with an alkyl iodide and potassium hydroxide in the presence of a tetrabutylammonium bromide catalyst was described, but the method required an expensive alkyl iodide and gave an yield of approximately 40%, which was not satisfied.

Next, in Step B, as a method to obtain compounds represented by the formula (1-2), a method to treat with dimethyl sulfate, after lithiating the 5-position of compounds represented by the formula (1-1) using butyl lithium in an ethereal solvent to treat with dimethyl sulfate is known in Liebigs Ann. Chem., vol. 625, 55 (1959). However, the yields were low, 1-alkylpyrazoles, i.e., the starting materials were remained in the reaction mixture and it was difficult for the desired 1-alkyl-5-methylpyrazoles represented by the formula (1-2) to be purely taken out.

Further, in Step C, in a method for nitrating the compounds represented by the formula (1-2), equivalent amounts of the acid and the nitrating agent, and the reaction temperature were not established and it was difficult to obtain the desired products stably in high yields.

### (2) Acetoacetic acid ester method

The acetoacetic acid ester method is a method for preparing 4-amino-5-methylpyrazoles represented by the formula (Ib) by reacting compounds represented by the formula (2-2) with compounds represented by the formula (2-3) to obtain compounds represented by the formula (2-4) (Step A), then converting the functional group of the compounds (2-4), and carrying out Hofmann rearrangement reaction, Schmidt rearrangement reaction, Curtius rearrangement reaction or Lossen rearrangement reaction (Step B).

### Step A and Step B:

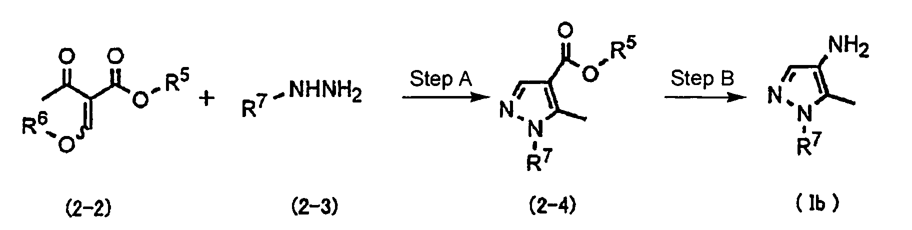

However, for example, in the reaction for forming the pyrazole ring by reacting the compounds represented by the formula (2-2) with the compounds represented by the formula (2-3) in Step A, it has been known that the 5-methylpyrazole-4-carboxylates are sometimes selectively obtained in a case where R⁷ is a phenyl group or a heteroaryl group, and it has also been known that selectivity in the reaction is low and the 3-methylpyrazole-4-carboxylates are sometimes produced more preferentially in a case where R⁷ is an alkyl group, an alkenyl group or an alkynyl group. Until now, as in Step B, there are no reports to synthesize the 4-amino-5-methylpyrazoles by Hofmann rearrangement reaction, Schmidt rearrangement reaction, Curtius rearrangement reaction or Lossen rearrangement reaction of the 5-methylpyrazole-4-carboxylic acid derivatives.

### (3) Acetylacetone method

The acetylacetone method is a method for preparing 4-amino-5-methylpyrazoles represented by the formula (Ic) by reacting compounds represented by the formula (3-2) with compounds represented by the formula (3-3) to obtain compounds represented by the formula (3-1) (Step A), then converting the compounds (3-1) to compounds (the hydroxyimino derivatives) represented by the formula (3-4) and carrying out Beckmann rearrangement reaction (Step B).

### Step A and Step B:

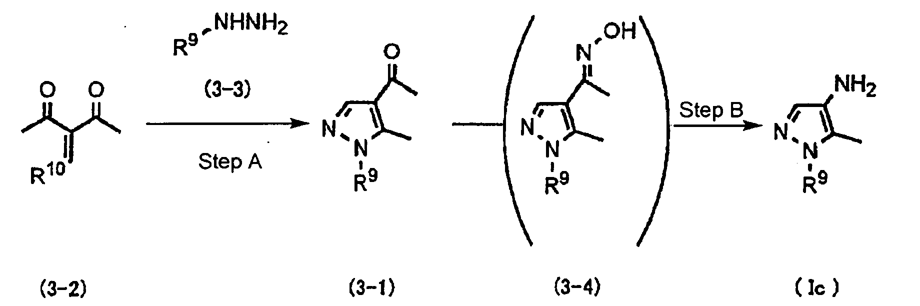

However, for example, in the reaction for forming the pyrazole ring by reacting the compounds represented by the formula (3-2) with the compounds represented by the formula (3-3) in Step A, it has been known that the 4-acetyl-5-methylpyrazoles are sometimes selectively obtained in a case where R⁹ is a phenyl group or a heteroaryl group, and it has been known that the selectivity in the reaction is low and the 4-acetyl-3-methylpyrazoles are sometimes produced more preferentially in a case where R⁹ is an alkyl group such as a methyl group. Further, there were no synthesis reports where R⁹ is an alkenyl group or an alkynyl group. Further, until now, there were no reports to synthesize the 4-amino-5-methylpyrazoles by Beckmann rearrangement reaction from the 4-acetyl-5-methylpyrazole derivatives as in Step B.

### Disclosure of invention

As described above, in the methods known until now, the 4-amino-5-methylpyrazoles have not been synthesized in high yields. The object of the present invention is to provide a means for synthesizing the 4-amino-5-methylpyrazoles being useful as synthetic intermediates of agricultural chemicals or the like in high yields by an industrially advantageous operation method.

The present inventors have intensively investigated to solve the above problem, and as a result, they have found the conditions or the like capable of synthesizing the 4-amino-5-methylpyrazoles in high yields in the respective methods such as the lithiation method, the acetoacetic acid ester method and the acetylacetone method as well as various novel compounds in the synthesis process, to accomplish the present invention based on these findings.

That is, the present invention includes the following inventions of (1) to (16).
(1) A 4-amino-5-methylpyrazole derivative represented by the following formula (I): 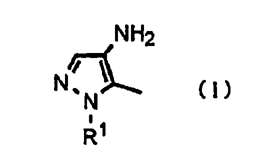 (wherein R¹ represents a group selected from group a, group b or group c <<provided that a methyl group is excluded>>, the group a is the group comprising a C₁-C₆ alkyl group which is the straight or branched alkyl group and may have optionally a C₃-C₆ alkyl group(s) as a substituent(s), the group b is the group comprising a straight or branched alkyl group which may have optionally a substituent(s), a straight or branched alkenyl group which is the alkenyl group bonded at the sp³ carbon atom and which may have optionally a substituent(s), a straight or branched alkynyl group which is the alkynyl group bonded at the sp³ carbon atom and which may have optionally a substituent(s) or a cycloalkenylmethyl group, and the group c is the group comprising a straight or branched alkyl group having 3 or more carbon chains which may have optinally a substituent(s), a cycloalkylmethyl group which may have optinally a substituent(s), a straight or branched alkenyl group which is the alkenyl group bonded at the sp³ carbon atom and which may have optinally a substituent(s), a straight or branched alkynyl group which is the alkynyl group bonded at the sp³ carbon atom and which may have optionally a substituent(s) or a cycloalkenylmethyl group)
   or a salt thereof.
(2) A process for preparing a 4-amino-5-methylpyrazole derivative represented by the following formula (Ia): 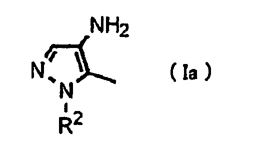 (wherein R² represents a C₁-C₆ alkyl group which is the straight or branched alkyl group and may have optionally a C₃-C₆ cycloalkyl group(s) as a substituent(s)), comprising a step for obtaining a compound represented by the following formula (1-1): 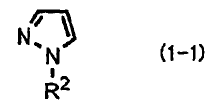 (wherein R² has the same meaning as defined above) by reacting 1H-pyrazole with a halogenated alkyl in two layers system of an aqueous sodium hydroxide solution and an organic solvent using a phase transfer catalyst.
(3) A process for preparing a 4-amino-5-methylpyrazole derivative represented by the following formula (Ia): 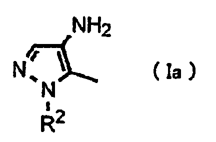 (wherein R² represents a C₁-C₆ alkyl group which is the straight or branched alkyl group and may have optionally a C₃-C₆ cycloalkyl group(s) as a substituent(s)),
   comprising a step for obtaining a compound represented by the following formula (1-2): 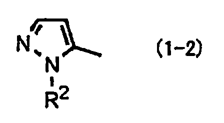 (wherein R² has the same meaning as defined above) by lithiating selectively the 5-position of the pyrazole ring of a compound represented by the following formula (1-1) : 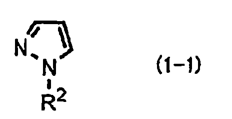 (wherein R² has the same meaning as defined above) in tetrahydrofuran using an alkyl lithium and reacting it with a methylating agent.
(4) A process for preparing a 4-amino-5-methylpyrazole derivative represented by the following formula (Ia): 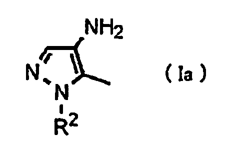 (wherein R² represents a C₁-C₆ alkyl group which is the straight or branched alkyl group and may have optionally a C₃-C₆ cycloalkyl group(s) as a substituent(s)),
   comprising nitrating a compound represented by the following formula (1-2): 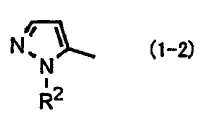 (wherein R² has the same meaning as defined above) and reducing it.
(5) A compound represented by the following formula (1-3): 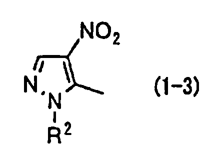 (wherein R² represents a C₁-C₆ alkyl group which is the straight or branched alkyl group and may have optionally a C₃-C₆ cycloalkyl group(s) as a substituent(s)).
(6) A compound represented by the following formula (2-1): 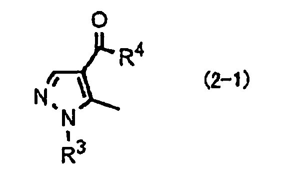 (wherein R³ represents a straight or branched alkenyl group which is the alkenyl group bonded at the sp³ carbon atom and may have optionally a substituent(s), a straight or branched alkynyl group which is the alkynyl group bonded at the sp³ carbon atom and may have optionally a substituent(s) or a cycloalkenylmethyl group; and R⁴ represents a hydroxyl group, a lower alkoxy group or an amino group).
(7) A process for preparing a compound represented by the following formula (2-4): 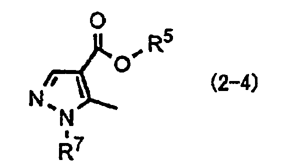 (wherein R⁵ represents a lower alkyl group and R⁷ represents a straight or branched alkyl group which may have optionally a substituent(s), a straight or branched alkenyl group which is the alkenyl group bonded at the sp³ carbon atom and may have optionally a substituent(s), a straight or branched alkynyl group which is the alkynyl group bonded at the sp³ carbon atom and may have optionally a substituent(s) or a cycloalkenylmethyl group),
   comprising reacting a compound represented by the following formula (2-2): 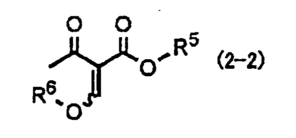 (wherein R⁵ has the same meaning as defined above and R⁶ independently represents a lower alkyl group)
   with a compound represented by the following formula (2-3) : 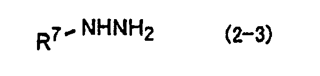 (wherein R⁷ has the same meaning as defined above)
   under co-existence of an acid.
(8) A compound represented by the following formula (2-4): 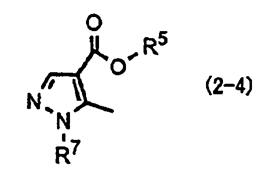 (wherein R⁵ represents a lower alkyl group and R⁷ represents a straight or branched alkyl group which may have optionally a substituent(s), a straight or branched alkenyl group which is the alkenyl group bonded at the sp³ carbon atom and may have optionally a substituent(s), a straight or branched alkynyl group which is the alkynyl group bonded at the sp³ carbon atom and may have optionally a substituent(s) or a cycloalkenylmethyl group).
(9) A process for preparing a compound represented by the following formula (2-5): 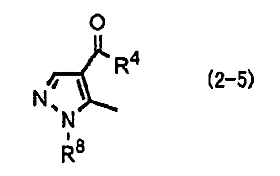 (wherein R⁴ represents a hydroxyl group, a lower alkoxy group or an amino group; and R⁸ represents a straight or branched alkyl group which may have optionally a substituent(s)),
   comprising hydrogenating a compound represented by the following formula (2-1): 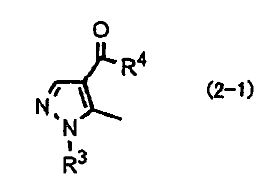 (wherein R³ represents a straight or branched alkenyl group which is the alkenyl group bonded at the sp³ carbon atom and may have optionally a substituent(s), a straight or branched alkynyl group which is the alkynyl group bonded at the sp³ carbon atom and may have optionally a substituent(s) or a cycloalkenylmethyl group; and R⁴ has the same meaning as defined above).
(10) A compound represented by the following formula (2-5) : 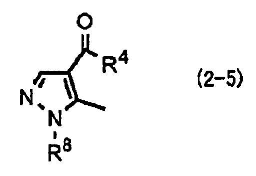 (wherein R⁴ represents a hydroxyl group, a lower alkoxy group or an amino group; and R⁸ represents a straight or branched alkyl group which may have optionally a substituent(s)).
(11) A process for preparing a 4-amino-5-methylpyrazole derivative represented by the formula (Ib),
   comprising obtaining the 4-amino-5-methylpyrazole derivative represented by the following formula (Ib): 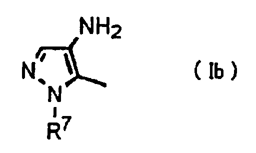 (wherein R⁷ represents a straight or branched alkyl group which may have optionally a substituent(s), a straight or branched alkenyl group which is the alkenyl group bonded at the sp³ carbon atom and may have optionally a substituent(s), a straight or branched alkynyl group which is the alkynyl group bonded at the sp³ carbon atom and may have optionally a substituent(s) or a cycloalkenylmethyl group)
   by converting the functional group of a compound represented by the following formula (2-4): 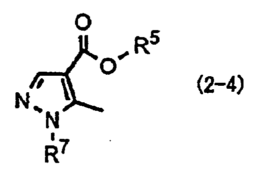 (wherein R⁵ represents a lower alkyl group; and R⁷ has the same meaning as defined above)
   and by carrying out Hofmann rearrangement reaction, Schmidt rearrangement reaction, Curtius rearrangement reaction or Lossen rearrangement reaction.
(12) A compound represented by the following formula (3-1) : 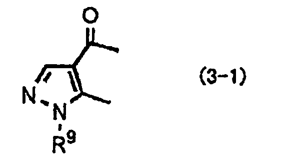 (wherein R⁹ represents a straight or branched alkyl group having 3 or more carbon chains which may have optionally a substituent(s), a cycloalkylmethyl group which may have optionally a substituent(s), a straight or branched alkenyl group which is the alkenyl group bonded at the sp³ carbon atom and may have optionally a substituent(s), a straight or branched alkynyl group which is the alkynyl group bonded at the sp³ carbon atom and may have optionally a substituent(s) or a cycloalkenylmethyl group).
(13) A process for preparing a compound represented by the following formula (3-1): 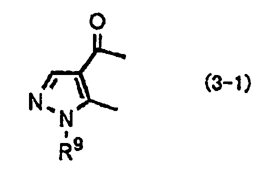 (wherein R⁹ represents a straight or branched alkyl group having 3 or more carbon chains which may have optionally a substituent(s), a cycloalkylmethyl group which may have optionally a substituent(s), a straight or branched alkenyl group which is the alkenyl group bonded at the sp³ carbon atom and may have optionally a substituent(s), a straight or branched alkynyl group which is the alkynyl group bonded at the sp³ carbon atom and may have optionally a substituent(s) or a cycloalkenylmethyl group),
   comprising reacting a compound represented by the following formula (3-2): 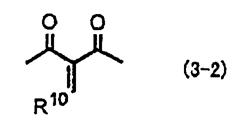 (wherein R¹⁰ represents a lower alkoxy group, a phenoxy group or a dialkylamino group)
   with a compound represented by the following formula (3-3) : 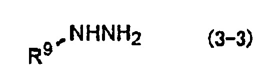 (wherein R⁹ has the same meaning as defined above)
   under co-existence of an acid.
(14) A process for preparing a compound represented by the following formula (3-1"): 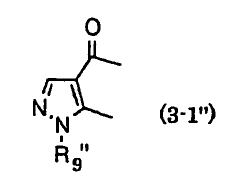 (wherein R₉" represents a straight or branched alkyl group having 3 or more carbon chains which may have optionally a substituent(s) or a cycloalkylmethyl group which may have optionally a substituent(s)),
   comprising hydrogenating a compound represented by the following formula (3-1'): 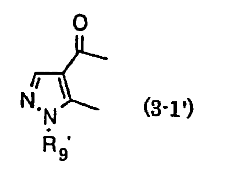 (wherein R₉' represents a straight or branched alkenyl group which is the alkenyl group bonded at the sp³ carbon atom and may have optionally a substituent(s), a straight or branched alkynyl group which is the alkynyl group bonded at the sp³ carbon atom and may have optionally a substituent(s) or a cycloalkenylmethyl group).
(15) A compound represented by the following formula (3-4) : 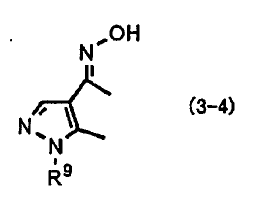 (wherein R⁹ represents a straight or branched alkyl group having 3 or more carbon chains which may have optionally a substituent(s), a cycloalkylmethyl group which may have optionally a substituent(s), a straight or branched alkenyl group which is the alkenyl group bonded at the sp³ carbon atom and may have optionally a substituent(s), a straight or branched alkynyl group which is the alkynyl group bonded at the sp³ carbon atom and may have optionally a substituent(s) or a cycloalkenylmethyl group).
(16) A process for preparing a 4-amino-5-methylpyrazole derivative represented by the formula (Ic),
   comprising obtaining the 4-amino-5-methylpyrazole derivative represented by the formula (Ic): 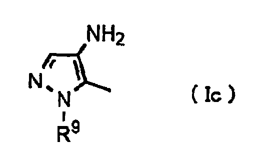 (wherein R⁹ represents a straight or branched alkyl group having 3 or more carbon chains which may have optionally a substituent(s), a cycloalkylmethyl group which may have optionally a substituent(s), a straight or branched alkenyl group which is the alkenyl group bonded at the sp³ carbon atom and may have optionally a substituent(s), a straight or branched alkynyl group which is the alkynyl group bonded at the sp³ carbon atom and may have optionally a substituent(s) or a cycloalkenylmethyl group) by carrying out Beckman rearrangement reaction from a compound represented by the following formula (3-4): 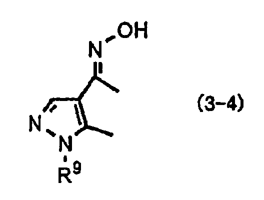 (wherein R₉ has the same meaning as defined above).

### [Best mode for carrying out invention]

In the following, the present invention is explained in more detail.

The present invention relates to 4-amino-5-methylpyrazole derivatives represented by the following formula (I): 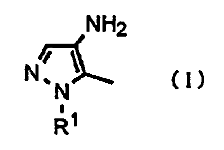 (wherein R¹ represents a group selected from group a, group b or group c <<provided that a methyl group is excluded>>, the group a is the group comprising a C₁-C₆ alkyl group which is the straight or branched alkyl group and may have optionally a C₃-C₆ alkyl group(s) as a substituent(s), the group b is the group comprising a straight or branched alkyl group which may have optionally a substituent(s), a straight or branched alkenyl group which is the alkenyl group bonded at the sp³ carbon atom and which may have optionally a substituent(s), a straight or branched alkynyl group which is the alkynyl group bonded the at sp³ carbon atom and which may have optionally a substituent(s) or a cycloalkenylmethyl group, and the group c is the group comprising a straight or branched alkyl group having from 3 or more carbon chains which may have optionally a substituent(s), a cycloalkylmethyl group which may have optionally a substituent(s), a straight or branched alkenyl group which is the alkenyl group bonded at the sp³ carbon atom and which may have optionally a substituent(s), a straight or branched alkynyl group which is the alkynyl group bonded at the sp³ carbon atom and which may have optionally a substituent(s) or a cycloalkenylmethyl group)
or a salt thereof.

When R¹ represents "a C₁-C₆ alkyl group which is the straight or branched alkyl group and may have optionally a C₃-C₆ alkyl group(s) as a substituent(s)" in the group a, "the straight or branched C₁-C₆ alkyl group" represents a methyl group, ethyl group, propyl group, isopropyl group, butyl group, isobutyl group, s-butyl group, t-butyl group, pentyl group, isopentyl group, s-pentyl group, t-pentyl group, neopentyl group and hexyl group, and preferably the methyl group, ethyl group, propyl group, isopropyl group, butyl group, isobutyl group, s-butyl group, pentyl group, isopentyl group, s-pentyl group, neopentyl group and hexyl group.

When R¹ represents "a C₁-C₆ alkyl group which is the straight or branched alkyl group and may have optionally a C₃-C₆ cycloalkyl group(s) as a substituent(s)" in the group a, "the C₃-C₆ cycloalkyl group" represents a cyclopropyl group, cyclobutyl group, cyclopentyl group and cyclohexyl group, and preferably the cyclobutyl group and cyclopentyl group.

When R¹ represents "a straight or branched alkyl group which may have optionally a substituent(s)" in the group b, it is the C₁-C₆ alkyl group. Such the alkyl group represents a methyl group, ethyl group, propyl group, isopropyl group, butyl group, isobutyl group, s-butyl group, t-butyl group, pentyl group, isopentyl group, s-pentyl group, t-pentyl group, neopentyl group and hexyl group, and preferably the methyl group, ethyl group, propyl group, isopropyl group, butyl group, isobutyl group, s-butyl group, pentyl group, isopentyl group, s-pentyl group, neopentyl group and hexyl group.

When R¹ represents "a straight or branched alkyl group which may have optionally a substituent(s)" in the group b, "the substituent group" represents a C₃-C₆ cycloalkyl group or a phenyl group. The C₃-C₆ cycloalkyl group is a cyclopropyl group, cyclobutyl group, cyclopentyl group or cyclohexyl group. The preferable substituent includes the cyclopentyl group, cyclobutyl group, cyclopentyl group and phenyl group, and more preferably the cyclobutyl group or phenyl group.

When R¹ represents "a straight or branched alkenyl group which is the alkenyl group bonded at the sp³ carbon atom and may have optionally a substituent(s)" in group b, "the alkenyl group" is a C₃-C₈ alkenyl group. Such the alkenyl group includes a 2-propenyl group, 1-methyl-2-propenyl group, 2-methyl-2-propenyl(metallyl) group, 2-ethyl-2-propenyl group, 2-butenyl group, 1-methyl-2-butenyl group, 2-methyl-2-butenyl group, 1-ethyl-2-butenyl group, 3-butenyl group, 1-methyl-3-butenyl group, 2-methyl-3-butenyl group, 1-ethyl-3-butenyl group, 2-pentenyl group, 1-methyl-2-pentenyl group, 2-methyl-2-pentenyl group, 3-pentenyl group, 1-methyl-3-pentenyl group, 2-methyl-3-pentenyl group, 4-pentenyl group, 1-methyl-4-pentenyl group, 2-methyl-4-pentenyl group, 5-hexenyl group, 6-heptenyl group and 7-octenyl group, preferably the 2-propenyl, 2-butenyl group, 2-methyl-2-propenyl(metallyl) group, 2-methyl-2-butenyl group, 2-pentenyl group, 3-pentenyl group and 4-pentenyl group, and more preferably the 2-propenyl, 2-butenyl group, 2-methyl-2-propenyl(metallyl) group and 2-methyl-2-butenyl group.

When R¹ represents "a straight or branched alkenyl group which is the alkenyl group bonded at the sp³ carbon atom and may have optionally a substituent(s)" in group b, "the substituent group" represents a phenyl group.

When R¹ represents "a straight or branched alkynyl group which is the alkenyl group bonded at the sp³ carbon atom and may have optionally a substituent(s)" in the group b, "the alkynyl group" is a C₃-C₈ alkynyl group. Such the alkynyl group includes a 2-propynyl group, 1-methyl-2-propynyl group, 2-butynyl group, 1-methyl-2-butynyl group, 1-ethyl-2-butynyl group, 3-butynyl group, 1-methyl-3-butynyl group, 2-methyl-3-butynyl group, 1-ethyl-3-butynyl group, 2-pentynyl group, 1-methyl-2-pentynyl group, 3-pentynyl group, 1-methyl-3-pentynyl group, 2-methyl-3-pentynyl group, 4-pentynyl group, 1-methyl-4-pentynyl group, 2-methyl-4-pentynyl group, 5-hexynyl group, 6-heptynyl group and 7-octynyl group, preferably the 2-propynyl group, 2-butynyl group, 3-butynyl group, 2-pentynyl group, 3-pentynyl group and 4-pentynyl group, and more preferably the 2-propynyl group, 2-butynyl group and 3-butynyl group.

When R¹ represents "a straight or branched alkynyl group which is the alkynyl group bonded at the sp³ carbon atom and may have optionally a substituent(s)" in the group b, "the substituent group" represents a phenyl group.

When R¹ represents "a cycloalkenylmethyl group" in the group b, "the cycloalkenyl group" represents a cyclopentenyl group or cyclohexenyl group.

When R¹ represents "a straight or branched alkyl group having 3 or more carbon chains which may have optionally a substituent(s)" in the group c, it is a C₃-C₆ alkyl group. Such the alkyl group includes a propyl group, isopropyl group, butyl group, isobutyl group, s-butyl group, t-butyl group, pentyl group, isopentyl group, s-pentyl group, t-pentyl group, neopentyl group and hexyl group, and preferably the propyl group, isopropyl group, butyl group, s-butyl group, pentyl group, isopentyl group, s-pentyl group, neopentyl group and hexyl group.

When R¹ represents "a straight or branched alkyl group having 3 or more carbon chains which may have optionally a substituent(s)" in the group c, "the substituent group" represents a phenyl group.

When R¹ represents "a cycloalkylmethyl group which may have optionally a substituent(s)" in the group c, "the cycloalkylmethyl group" includes a cyclopropylmethyl group, cyclobutylmethyl group, cyclopentylmethyl group and cyclohexylmethyl group, and preferably the cyclopropylmethyl group, cyclobutylmethyl group and cyclopentylmethyl group.

When R¹ represents "a cycloalkylmethyl group which may have optionally a substituent(s)" in the group c, "the substituent group" represents a lower alkyl group or phenyl group. The lower alkyl group includes, for example, a methyl group, ethyl group, propyl group, isopropyl group, butyl group and t-butyl group. The preferable substituent includes the methyl group, ethyl group or phenyl group, and more preferably the methyl group or phenyl group.

When R¹ represents "a straight or branched alkenyl group which is the alkenyl group bonded at the sp³ carbon atom and may have optionally a substituent(s)" in the group c, "the alkenyl group is a C₃-C₈ alkenyl group. Such the alkenyl group includes a 2-propenyl group, 1-methyl-2-propenyl group, 2-methyl-2-propenyl(metallyl) group, 2-ethyl-2-propenyl group, 2-butenyl group, 1-methyl-2-butenyl group, 2-methyl-2-butenyl group, 1-ethyl-2-butenyl group, 3-butenyl group, 1-methyl-3-butenyl group, 2-methyl-3-butenyl group, 1-ethyl-3-butenyl group, 2-pentenyl group, 1-methyl-2-pentenyl group, 2-methyl-2-pentenyl group, 3-pentenyl group, 1-methyl-3-pentenyl group, 2-methyl-3-pentenyl group, 4-pentenyl group, 1-methyl-4-pentenyl group, 2-methyl-4-pentenyl group, 5-hexenyl group, 6-heptenyl group and 7-octenyl group, preferably the 2-propenyl, 2-butenyl group, 2-methyl-2-propenyl(metallyl) group, 2-methyl-2-butenyl group, 2-pentenyl group, 3-pentenyl group and 4-pentenyl group, and more preferably the 2-propenyl, 2-butenyl group, 2-methyl-2-propenyl(metallyl) group and 2-methyl-2-butenyl group.

When R¹ represents "a straight or branched alkenyl group which is the alkenyl group bonded at the sp³ carbon atom and may have optionally a substituent(s)" in the group c, "the substituent group" represents a phenyl group.

When R¹ represents "a straight or branched alkynyl group which is the alkynyl group bonded at the sp³ carbon atom and may have optionally a substituent(s)" in the group c, "the alkynyl group" is a C₃-C₈ alkynyl group. Such the alkynyl group includes a 2-propynyl group, 1-methyl-2-propynyl group, 2-butynyl group, 1-methyl-2-butynyl group, 1-ethyl-2-butynyl group, 3-butynyl group, 1-methyl-3-butynyl group, 2-methyl-3-butynyl group, 1-ethyl-3-butynyl group, 2-pentynyl group, 1-methyl-2-pentynyl group, 3-pentynyl group, 1-methyl-3-pentynyl group, 2-methyl-3-pentynyl group, 4-pentynyl group, 1-methyl-4-pentynyl group, 2-methyl-4-pentynyl group, 5-hexynyl group, 6-heptynyl group and 7-octynyl group, preferably the 2-propynyl group, 2-butynyl group, 3-butynyl group, 2-pentynyl group, 3-pentynyl group and 4-pentynyl group, and more preferably the 2-propynyl group, 2-butynyl group and 3-butynyl group.

When R¹ represents "a straight or branched alkynyl group which is the alkynyl group bonded at the sp³ carbon atom and may have optionally a substituent(s)" in the group c, "the substituent group" represents a phenyl group.

When R¹ represents "a cycloalkenylmethyl group" in the group c, the cycloalkenyl group represents a cyclopentenyl group or cyclohexenyl group.

Further, in the present invention, "a salt of the 4-amino-5-methylpyrazole derivatives" may include, for example, a hydrochloride, hydrobromide, sulfate, nitrate, phosphate, formate, acetate, methanesulfonate, p-toluenesulfonate, trifluoroacetate, trichloroacetate and trifluoromethanesulfonate.

"The 4-amino-5-methylpyrazole derivatives" represented by the formula (I) can be prepared by (1) lithiation method, (2) acetoacetic acid ester method and (3) acetylacetone method shown below. In the following, the respective methods are explained.

### (1) Lithiation method

The lithiation method of the present invention includes Step A and Step B described later. In this method, the compounds represented by the following formulae (1-1) and (1-2) are produced as synthetic intermediates and the final products are compounds represented by the formula (Ia): 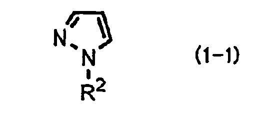 (wherein R² represents a C₁-C₆ alkyl group which is the straight or branched alkyl group and may have a C₃-C₆ cycloalkyl(s) as a substituent(s)), 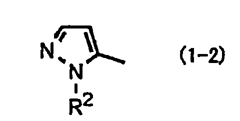 (wherein R² has the same meaning as defined above) and 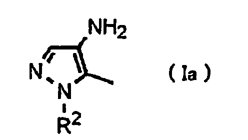 (wherein R² has the same meaning as defined above).

When R² represents "a C₁-C₆ alkyl group which is the straight or branched alkyl group and may have optionally a C₃-C₆ cycloalkyl(s) as a substituent(s)", "the straight or branched C₁-C₆ alkyl group" represents a methyl group, ethyl group, propyl group, isopropyl group, butyl group, isobutyl group, s-butyl group, t-butyl group, pentyl group, isopentyl group, s-pentyl group, t-pentyl group, neopentyl group and hexyl group, and preferably the methyl group, ethyl group, propyl group, isopropyl group, butyl group, isobutyl group, s-butyl group, pentyl group, isopentyl group, s-pentyl group, neopentyl group and hexyl group.

When R² represents "a C₁-C₆ alkyl group which is the straight or branched alkyl group and may have optionally a C₃-C₆ cycloalkyl(s) as a substituent(s)", "the C₃-C₆ cycloalkyl group" represents a cyclopropyl group, cyclobutyl group, cyclopentyl group and cyclohexyl group, and preferably the cyclobutyl group and cyclopentyl group.

Step A in the lithiation method of the present invention is to prepare the 1-alkylpyrazoles represented by the formula (1-1) by reacting 1H-pyrazole with an halogenated alkyl in a two layers system of an aqueous sodium hydroxide solution and an organic solvent using a phase transfer catalyst.

### Step A:

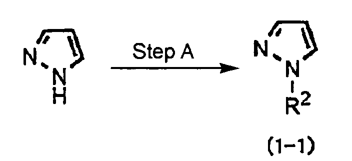

The phase transfer catalyst used in the present step is not limited so long as it has phase transfer catalytic ability. Such the catalyst includes, for example, quaternary ammonium salts such as tetramethylammonium salts, tetraethylammonium salts, tetrabutylammonium salts, benzyltrimethylammonium salts, Aliquat (registered trademark) 175 or Aliquat (registered trademark) 336; phosphonium salts such as tetramethylphosphonium salts, tetrabutylphosphonium salts or methyltriphenylphosphonium salts; or crown ethers, preferably the ammonium salts, and more preferably the tetrabutylammonium bromide, tetrabutylammonium sulfate or Aliquat (registered trademark) 336.

An amount of sodium hydroxide used in the present step is not particularly limited so long as it is 1 equivalent or more relative to 1H-pyrazole, and is preferably from 2 to 4 equivalents and more preferably from 2 to 3 equivalents.

Concentration of the aqueous sodium hydroxide solution used in the present step is not particularly limited, and is preferably from 10% to a saturated solution and more preferably from 30% to a saturated solution.

The halogenated alkyl used in the present step is, for example, an alkyl chloride or alkyl bromide.

An amount of the halogenated alkyl used in the present step is not particularly limited so long as it is 1 equivalent or more relative to 1H-pyrazole, and is preferably from 1 to 2 equivalents and more preferably from 1 to 1.2 equivalents.

The organic solvent used in the present step is not particularly limited so long as it is inactive for the reaction, and includes preferably hydrocarbons such as hexane, octane, decane, isooctane, cyclohexane, methylcyclohexane, dimethylcyclohexane, benzene, toluene, xylene and mesitylene, and more preferably methylcyclohexane, dimethylcyclohexane, toluene and xylene.

The reaction temperature is not particularly limited, and is usually from room temperature to reflux temperature, and preferably from 60°C to reflux temperature.

Step B in the lithiation method of the present invention is to prepare the 1-alkyl-5-methylpyrazoles represented by the formula (1-2) by lithiating selectively the 5-position of the 1-alkylpyrazoles represented by the formula (1-1) in tetrahydrofuran using an alkyl lithium and reacting with a methylating agent.

### Step B:

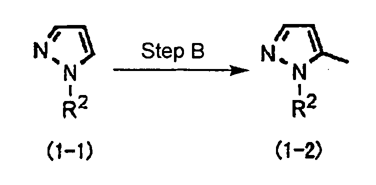

The alkyl lithium used in the present step includes, for example, methyl lithium, ethyl lithium, propyl lithium, isopropyl lithium, butyl lithium, s-butyl lithium, t-butyl lithium or the like, and preferably butyl lithium.

An amount of the alkyl lithium used in the present step is usually from 1 to 1.5 equivalents relative to the 1-alkyl-5-methylpyrazoles represented by the formula (1-2) used, preferably from 1 to 1.3 equivalents, and more preferably from 1 to 1.2 equivalents.

The methylating agent used in the present step is not particularly limited so long as it is an electrophilic methylating agent usually used. Such the methylating agent includes, for example, methyl iodide, methyl bromide, dimethyl sulfate, methyl trifluoromethanesulfonate and dimethyl carbonate, preferably methyl iodide, dimethyl sulfate and methyl trifluoromethanesulfonate, and more preferably methyl iodide.

An amount of the methylating agent used in the present step has no problem so long as it is 1 equivalent or more relative to the 1-alkylpyrazoles represented by the formula (1-1) used, and is preferably from 1 to 1.5 equivalents, and more preferably from 1 to 1.2 equivalents.

The reaction temperature of the present step is usually from -100°C to -10°C, preferably from -70°C to -10°C, and more preferably from -30°C to -10°C.

Step C in the lithiation method of the present invention is to prepare the 4-amino-1-alkyl-5-methylpyrazoles represented by the formula (Ia) by nitrating selectively the 4-position of the 1-alkyl-5-methylpyrazoles represented by the formula (1-2) to obtain the 1-alkyl-4-nitro-5-methylpyrazoles represented by the formula (1-3) and reducing the nitro group, and comprises a step of nitration (Step C1) and a step of reduction (Step C2).

### Step C:

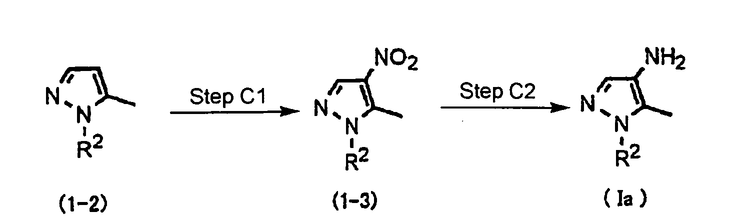

Step C1 is usually carried out using a nitrating agent in concentrated sulfuric acid.

The nitrating agent used in the present step is not particularly limited so long as it is usually used in the nitration under an acidic condition. Such the nitrating agent includes, for example, concentrated nitric acid, sodium nitrate, potassium nitrate and ammonium nitrate, and preferably concentrated nitric acid.

An amount of the nitrating agent used in the present step is usually form 1 to 3 equivalents relative to the 1-alkyl-5-methylpyrazoles represented by the formula (1-2) used, preferably form 1 to 2 equivalents, and more preferably from 1 to 1.6 equivalents.

An amount of concentrated sulfuric acid used in the present step is usually from 6 to 12 equivalents relative to the 1-alkyl-5-methylpyrazoles represented by the formula (1-2), preferably from 6 to 10 equivalents, and more preferably from 6.5 to 8 equivalents.

The reaction temperature is not particularly limited so long as it is from 0°C to 150°C and the reaction is preferably started at from 10°C to room temperature and the nitrating agent is added so as to maintain the temperature at from 30°C to 70°C by the heat of the reaction .

After completion of the reaction, the mixture is neutralized and extracted to obtain the desired compounds, and an organic base is usually used as a neutralizing agent. Such the organic base includes, for example, sodium hydroxide, potassium hydroxide, sodium carbonate, potassium carbonate, sodium hydrogencarbonate, potassium hydrogencarbonate and aqueous ammonia, preferably sodium hydroxide or aqueous ammonia, and more preferably aqueous ammonia.

Step C2 is usually carried out by catalytic hydrogenation.

The catalyst used in the present step is not particularly limited so long as it is usually used for catalytic halogenation. Such the catalyst used includes, for example, a palladium-carbon catalyst, platinum-carbon catalyst, Raney nickel, Wilkinson complex or the like , preferably the palladium-carbon catalyst or Raney nickel, and more preferably the palladium-carbon catalyst.

Pressure of hydrogen in the present step is not particularly limited so long as it is 1 atm or more and is usually from 1 to 20 atm, and preferably from 1 to 10 atm.

The present step is usually carried out in a solvent. The solvent is not particularly limited so long as it is inactive for the present reaction and such a solvent includes, for example, water; alcohols such as methanol, ethanol, propanol, or isopropyl alcohol; or esters such as methyl acetate, ethyl acetate or butyl acetate, preferably water, methanol or ethyl acetate, more preferably methanol.

The reaction temperature of the present step is not particularly limited, and the present step is usually carried out at from room temperature to 150°C, and preferably the reaction is started at room temperature and the pressure of hydrogen is adjusted so as to maintain the temperature at from 40°C to 130°C by the heat of the reaction.

After completion of reaction of the respective steps of Step A, Step B and Step C, the mixture is made to acidic, neutral or basic corresponding to a physical property of the products after post-treatment and thereafter isolation operation is carried out. After the isolation, the product can be used in the subsequent step as such or, if necessary, with conventional purifications such as distillation, recrystalization or chromatography.

### (2) Acetoacetic acid ester method

The acetoacetic acid ester method of the present invention includes Step A and Step B described later and optionally includes further Step C. In this method, the compounds represented by the following formulae (2-1), (2-2), (2-3), (2-4) and (2-5) are produced as synthetic intermediates and the final products are the compounds represented by the formula (Ib). 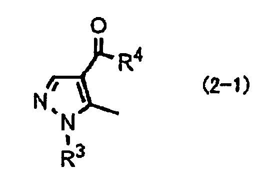 (wherein R³ represents a straight or branched alkenyl group which is the alkenyl group bonded at the sp³ carbon atom and may have optionally a substituent(s), a straight or branched alkynyl group which is the alkynyl group bonded at the sp³ carbon atom and may have optionally a substituent(s) or a cycloalkenylmethyl group; and R⁴ represents a hydroxyl group, a lower alkoxy group or an amino group); 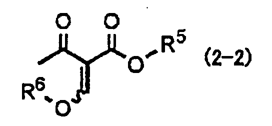 (wherein R⁵ and R⁶ each independently represents a lower alkyl group); 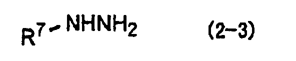 (wherein R⁷ represents a straight or branched alkyl group which may have optionally a substituent(s), a straight or branched alkenyl group which is the alkenyl group bonded at the sp³ carbon atom and may have optionally a substituent(s), a straight or branched alkynyl group which is the alkynyl group bonded at the sp³ carbon atom and may have optionally a substituent(s) or a cycloalkenylmethyl group); 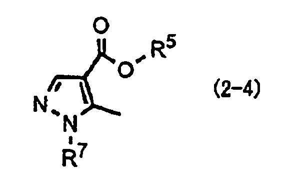 (wherein R⁵ and R⁷ have the same meanings as defined above); 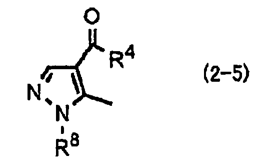 (wherein R⁸ represents an alkyl group which may have optionally a substituent(s)); and 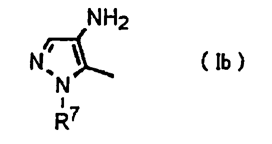 (wherein R⁷ has the same meaning as defined above).

When R³ represents "a straight or branched alkenyl group which is the alkenyl group bonded at the sp³ carbon atom and may have optionally a substituent(s)", "the alkenyl group" is a C₃-C₈ alkenyl group. Such the alkenyl group includes a 2-propenyl group, 1-methyl-2-propenyl group, 2-methyl-2-propenyl(metallyl) group, 2-ethyl-2-propenyl group, 2-butenyl group, 1-methyl-2-butenyl group, 2-methyl-2-butenyl group, 1-ethyl-2-butenyl group, 3-butenyl group, 1-methyl-3-butenyl group, 2-methyl-3-butenyl group, 1-ethyl-3-butenyl group, 2-pentenyl group, 1-methyl-2-pentenyl group, 2-methyl-2-pentenyl group, 3-pentenyl group, 1-methyl-3-pentenyl group, 2-methyl-3-pentenyl group, 4-pentenyl group, 1-methyl-4-pentenyl group, 2-methyl-4-pentenyl group, 5-hexenyl group, 6-heptenyl group and 7-octenyl group, preferably the 2-propenyl, 2-butenyl group, 2-methyl-2-propenyl(metallyl) group, 2-methyl-2-butenyl group, 2-pentenyl group, 3-pentenyl group and 4-pentenyl group, and more preferably the 2-propenyl, 2-butenyl group, 2-methyl-2-propenyl(metallyl) group and 2-methyl-2-butenyl group.

When R³ represents "a straight or branched alkenyl group which is the alkenyl group bonded at the sp³ carbon atom and may have optionally asubstituent(s)", "the substituent" represents a phenyl group.

When R³ represents "a straight or branched alkynyl group which is the alkynyl group bonded at the sp³ carbon atom and may have optionally a substituent(s)", "the alkynyl group" is a C₃ - C₈ alkynyl group. Such the alkynyl group includes a 2-propynyl group, 1-methyl-2-propynyl group, 2-butynyl group, 1-methyl-2-butynyl group, 1-ethyl-2-butynyl group, 3-butynyl group, 1-methyl-3-butynyl group, 2-methyl-3-butynyl group, 1-ethyl-3-butynyl group, 2-pentynyl group, 1-methyl-2-pentynyl group, 3-pentynyl group, 1-methyl-3-pentynyl group, 2-methyl-3-pentynyl group, 4-pentynyl group, 1-methyl-4-pentynyl group, 2-methyl-4-pentynyl group, 5-hexynyl group, 6-heptynyl group and 7-octynyl group, preferably the 2-propynyl group, 2-butynyl group, 3-butynyl group, 2-pentynyl group, 3-pentynyl group and 4-pentynyl group, and more preferably the 2-propynyl group, 2-butynyl group and 3-butynyl group.

When R³ represents "a straight or branched alkynyl group which is the alkynyl group bonded at the sp³ carbon atom and may have optionally a substituent(s)", "the substituent" represents a phenyl group.

When R³ represents "a cycloalkenylmethyl group", "the cycloalkenyl group" represents a cyclopentenyl group or cyclohexenyl group.

When R⁴ represents "a lower alkoxy group", "the lower alkoxy group" is a straight or branched C₁-C₄ alkoxy group. Such the alkoxy group includes, for example, a methoxy group, ethoxy group, propoxy group, isopropoxy group, butoxy group, isobutoxy group or t-butoxy group, preferably the methoxy group, ethoxy group or t-butoxy group, and more preferably the methoxy group or ethoxy group.

When R⁵ and R⁶ each independently represents "a lower alkyl group", they are may be the same or different each other. Such the lower alkyl group includes, for example, a methyl group, ethyl group, propyl group, isopropyl group, butyl group and t-butyl group, preferably the methyl group, ethyl group or t-butyl group, and more preferably the methyl group or ethyl group.

When R⁷ represents "a straight or branched alkyl group which may have optionally a substituent(s)", it is a C₁-C₆ alkyl group. Such the alkyl group represents a methyl group, ethyl group, propyl group, isopropyl group, butyl group, isobutyl group, s-butyl group, t-butyl group, pentyl group, isopentyl group, s-pentyl group, t-pentyl group, neopentyl group and hexyl group, and preferably the methyl group, ethyl group, propyl group, isopropyl group, butyl group, isobutyl group, s-butyl group, pentyl group, isopentyl group, s-pentyl group, neopentyl group and hexyl group.

When R⁷ represents "a straight or branched alkyl group which may have a substituent(s)", "the substituent" represents a C₃-C₆ cycloalkyl group or a phenyl group. The C₃-C₆ cycloalkyl group includes a cyclopropyl group, cyclobutyl group, cyclopentyl group or cyclohexyl group. The preferable substituent includes the cyclopentyl group, cyclobutyl group, cyclopentyl group and phenyl group, and more preferably the cyclobutyl group or phenyl group.

When R⁷ represents "a straight or branched alkenyl group which is the alkenyl group bonded at the sp³ carbon atom and may have optionally a substituent(s)", "the alkenyl group" is a C₃-C₈ alkenyl group. Such the alkenyl group includes a 2-propenyl group, 1-methyl-2-propenyl group, 2-methyl-2-propenyl(metallyl) group, 2-ethyl-2-propenyl group, 2-butenyl group, 1-methyl-2-butenyl group, 2-methyl-2-butenyl group, 1-ethyl-2-butenyl group, 3-butenyl group, 1-methyl-3-butenyl group, 2-methyl-3-butenyl group, 1-ethyl-3-butenyl group, 2-pentenyl group, 1-methyl-2-pentenyl group, 2-methyl-2-pentenyl group, 3-pentenyl group, 1-methyl-3-pentenyl group, 2-methyl-3-pentenyl group, 4-pentenyl group, 1-methyl-4-pentenyl group, 2-methyl-4-pentenyl group, 5-hexenyl group, 6-heptenyl group and 7-octenyl group, preferably the 2-propenyl, 2-butenyl group, 2-methyl-2-propenyl(matallyl) group, 2-methyl-2-butenyl group, 2-pentenyl group, 3-pentenyl group and 4-pentenyl group, and more preferably the 2-propenyl, 2-butenyl group, 2-methyl-2-propenyl(metallyl) group and 2-methyl-2-butenyl group.

When R⁷ represents."a straight or branched alkenyl group which is the alkenyl group bonded at the sp³ carbon atom and may have optionally a substituent(s)", "the substituent" represents a phenyl group.

When R⁷ represents "a straight or branched alkynyl group which is the alkynyl group bonded at the sp³ carbon atom and may have optionally a substituent(s)", "the alkynyl group" is a C₃-C₈ alkynyl group. Such the alkynyl group includes a 2-propynyl group, 1-methyl-2-propynyl group, 2-butynyl group, 1-methyl-2-butynyl group, 1-ethyl-2-butynyl group, 3-butynyl group, 1-methyl-3-butynyl group, 2-methyl-3-butynyl group, 1-ethyl-3-butynyl group, 2-pentynyl group, 1-methyl-2-pentynyl group, 3-pentynyl group, 1-methyl-3-pentynyl group, 2-methyl-3-pentynyl group, 4-pentynyl group, 1-methyl-4-pentynyl group, 2-methyl-4-pentynyl group, 5-hexynyl group, 6-heptynyl group and 7-octynyl group, preferably the 2-propynyl group, 2-butynyl group, 3-butynyl group, 2-pentynyl group, 3-pentynyl group and 4-pentynyl group, and more preferably the 2-propynyl group, 2-butynyl group and 3-butynyl group.

When R⁷ represents "a straight or branched alkynyl group which is the alkynyl group bonded at the sp³ carbon atom and may have optionally a substituent(s)", "the substituent" represents a phenyl group.

When R⁸ represents "a straight or branched alkyl group which may have optionally a substituent(s)", it is a C₁-C₆ alkyl groups. Such the alkyl group represents a methyl group, ethyl group, propyl group, isopropyl group, butyl group, isobutyl group, s-butyl group, t-butyl group, pentyl group, isopentyl group, s-pentyl group, t-pentyl group, neopentyl group and hexyl group, and preferably the methyl group, ethyl group, propyl group, isopropyl group, butyl group, isobutyl group, s-butyl group, pentyl group, isopentyl group, s-pentyl group, neopentyl group and hexyl group.

When R⁸ represents "an alkyl group which may have optionally a substituent(s)", "the substituent" represents a C₃-C₆ cycloalkyl group or a phenyl group. The C₃-C₆ cycloalkyl group includes a cyclopropyl group, cyclobutyl group, cyclopentyl group or cyclohexyl group. The preferable substituent includes the cyclopentyl group, cyclobutyl group, cyclopentyl group and phenyl group, and more preferably the cyclobutyl group or phenyl group.

Exemplified compounds of the compounds represented by the formula (2-1) of the present invention are shown in Table 1 and Table 2.

**[Table 1]**

| **R**^{**3**} | **R**^{**4**} |
|---|---|
| **-CH**_{**2**}**CHCH**_{**2**} | **OH** |
| **-CH**_{**2**}**CHCH**_{**2**} | **OMe** |
| **-CH**_{**2**}**-CHCH**_{**2**} | **OEt** |
| **-CH**_{**2**}**CHCH**_{**2**} | **OPr** |
| **-CH**_{**2**}**CHCH**_{**2**} | **OiPr** |
| **-CH**_{**2**}**CHCH**_{**2**} | **OBu** |
| **-CH**_{**2**}**CHCH**_{**2**} | **NH**_{**2**} |
| **-CH**_{**2**}**CHCHCH**_{**3**} | **OH** |
| **CH**_{**2**}**CHCHCH**_{**3**} | **OMe** |
| **CH**_{**2**}**CHCHCH**_{**3**} | **OEt** |
| **CH**_{**2**}**CHCHCH**_{**3**} | **OPr** |
| **CH**_{**2**}**CHCHCH**_{**3**} | **OiPr** |
| **CH**_{**2**}**CHCHCH**_{**3**} | **OBu** |
| **CH**_{**2**}**CHCHCH**_{**3**} | **NH**_{**2**} |
| **CH**_{**2**}**CHC(CH**_{**3**}**)**_{**2**} | **OH** |
| **CH**_{**2**}**CHC(CH**_{**3**}**)**_{**2**} | **OMe** |
| **CH**_{**2**}**CHC(CH**_{**3**}**)**_{**2**} | **OEt** |
| **CH**_{**2**}**CHC(CH**_{**3**}**)**_{**2**} | **OPr** |
| **CH**_{**2**}**CHC(CH**_{**3**}**)**_{**2**} | **OiPr** |
| **CH**_{**2**}**CHC(CH**_{**3**}**)**_{**2**} | **OBu** |
| **CH**_{**2**}**CHC(CH**_{**3**}**)**_{**2**} | **NH**_{**2**} |
| **-CH**_{**2**}**C(CH**_{**3**}**)CH**_{**2**} | **OH** |
| **-CH**_{**2**}**C(CH**_{**3**}**)CH**_{**2**} | **OMe** |
| **-CH**_{**2**}**C(CH**_{**3**}**)CH**_{**2**} | **OEt** |
| **-CH**_{**2**}**C(CH**_{**3**}**)CH**_{**2**} | **OPr** |
| **-CH**_{**3**}**C(CH**_{**3**}**)CH**_{**2**} | **OiPr** |
| **-CH**_{**2**}**C(CH**_{**3**}**)CH**_{**2**} | **OBu** |
| **-CH**_{**2**}**C(CH**_{**3**}**)CH**_{**2**} | **NH**_{**2**} |
| **-CH**_{**2**}**C(CH**_{**3**}**)CHCH**_{**3**} | **OH** |
| **-CH**_{**2**}**C(CH**_{**3**}**)CHCH**_{**3**} | **OMe** |
| **-CH**_{**2**}**C(CH**_{**3**}**)CHCH**_{**3**} | **OEt** |
| **-CH**_{**2**}**C(CH**_{**3**}**)CHCH**_{**3**} | **OPr** |
| **-CH**_{**2**}**C(CH**_{**3**}**)CHCH**_{**3**} | **OiPr** |
| **-CH**_{**2**}**C(CH**_{**3**}**)CHCH**_{**3**} | **OBu** |
| **-CH**_{**2**}**C(CH**_{**3**}**)CHCH**_{**3**} | **NH**_{**2**} |
| **-CH**_{**2**}**C(CH**_{**3**}**)C(CH**_{**3**}**)**_{**2**} | **OH** |
| **-CH**_{**2**}**C(CH**_{**3**}**)C(CH**_{**3**}**)**_{**2**} | **OMe** |
| **-CH**_{**2**}**C(CH**_{**3**}**)C(CH**_{**3**}**)**_{**2**} | **OEt** |
| **-CH**_{**2**}**C(CH**_{**3**}**)C(CH**_{**3**}**)**_{**2**} | **OPr** |
| **-CH**_{**2**}**C(CH**_{**3**}**)C(CH**_{**3**}**)**_{**2**} | **OiPr** |
| **-CH**_{**2**}**C(CH**_{**3**}**)C(CH**_{**3**}**)**_{**2**} | **OBu** |
| **-CH**_{**2**}**C(CH**_{**3**}**)C(CH**_{**3**}**)**_{**2**} | **NH**_{**2**} |

**[Table 2]**

| **R**^{**3**} | **R**^{**4**} |
|---|---|
| **-CH**_{**2**}**-cycPen** | **OH** |
| **-CH**_{**2**}**-cycPen** | **OMe** |
| **-CH**_{**2**}**-cycPen** | **OEt** |
| **-CH**_{**2**}**-cycPen** | **OPr** |
| **-CH**_{**2**}**-cycPen** | **OiPr** |
| **-CH**_{**2**}**-cycPen** | **OBu** |
| **-CH**_{**2**}**-cycPen** | **NH**_{**2**} |
| **-CH**_{**2**}**-cycHex** | **OH** |
| **-CH**_{**2**}**-cycHex** | **OMe** |
| **-CH**_{**2**}**-cycHex** | **OEt** |
| **-CH**_{**2**}**-cycHex** | **OPr** |
| **-CH**_{**2**}**-cycHex** | **OiPr** |
| **-CH**_{**2**}**-cycHex** | **OBu** |
| **-CH**_{**2**}**-cycHex** | **NH**_{**2**} |
| **-CH**_{**2**}**CCH** | **OH** |
| **-CH**_{**2**}**CCH** | **OMe** |
| **-CH**_{**2**}**CCH** | **OEt** |
| **-CH**_{**2**}**CCH** | **OPr** |
| **-CH**_{**2**}**CCH** | **OiPr** |
| **-CH**_{**2**}**CCH** | **OBu** |
| **-CH**_{**2**}**CCH** | **NH**_{**2**} |
| **-CH**_{**2**}**-CCCH**_{**3**} | **OH** |
| **-CH**_{**2**}**-CCCH**_{**3**} | **OMe** |
| **-CH**_{**2**}**-CCCH**_{**3**} | **OEt** |
| **-CH**_{**2**}**-CCCH**_{**3**} | **OPr** |
| **-CH**_{**2**}**-CCCH**_{**3**} | **OiPr** |
| **-CH**_{**2**}**-CCCH**_{**3**} | **OBu** |
| **-CH**_{**2**}**-CCCH**_{**3**} | **NH**_{**2**} |

In the Tables, Me represents a methyl group, Et represents an ethyl group, Pr represents a propyl group, iPr represents an isopropyl group, Bu represents a butyl group, cycPen represents a 1-cyclopentenyl group and cycHex represents a 1-cyclohexenyl group, respectively.

Step A of the acetoacetic acid ester method of the present invention is to prepare the compounds represented by the formula (2-4) regionselectively by reacting the compounds represented by the formula (2-2) with the compounds represented by the formula (2-3) under co-existence of an acid.

### Step A:

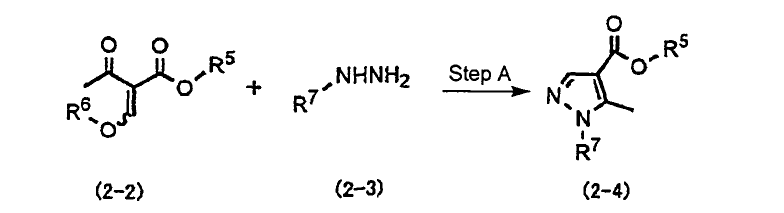

An acid used in the present step is not particularly limited so long as it is an usual Bronsted acid. Such the acid includes, for example, hydrogen chloride, hydrochloric acid, hydrobromic acid, hydroiodic acid, sulfuric acid, nitric acid, methanesulfonic acid, p-toluenesulfonic acid, formic acid, acetic acid or a mixture thereof, preferably hydrogen chloride, hydrochloric acid, sulfuric acid, methanesulfonic acid or p-toluenesulfonic acid, and more preferably hydrogen chloride, hydrochloric acid or sulfuric acid.

An amount of the acid used is not particularly limited so long as it is 1 equivalent or more relative to the compounds of the above formula (2-3) and is usually from 1 to 10 equivalents, preferably from 1 to 4 equivalents, and more preferably from 1 to 1.5 equivalents.

The acid used may be added separately from the compounds of the above formula (2-3) and may be used as a salt of the compounds of the formula (2-3).

The reaction is usually carried out in a solvent. The solvent is not particularly limited so long as it does not inhibit the reaction. Such the solvent includes, for example, water; alcohols such as methanol, ethanol, propanol, isopropyl alcohol or butanol; ethers such as diethyl ether or tetrahydrofuran; or halogenated solvents such as dichloromethane, chloroform or 1,2-dichloroethane, preferably the alcohols, and more preferably methanol or ethanol.

The reaction temperature is not particularly limited and the reaction is usually carried out at from -70°C to 50°C, preferably -20°C to room temperature, and more preferably -5°C to 10°C.

Step B of the acetoacetic acid ester method of the present invention is to prepare the 4-amino-5-methylpyrazole derivatives represented by the formula (Ib) by converting the functional group of the compounds represented by the formula (2-4) and carrying out Hofmann rearrangement reaction, Schmidt rearrangement reaction, Curtius rearrangement reaction or Lossen rearrangement reaction.

### Step B:

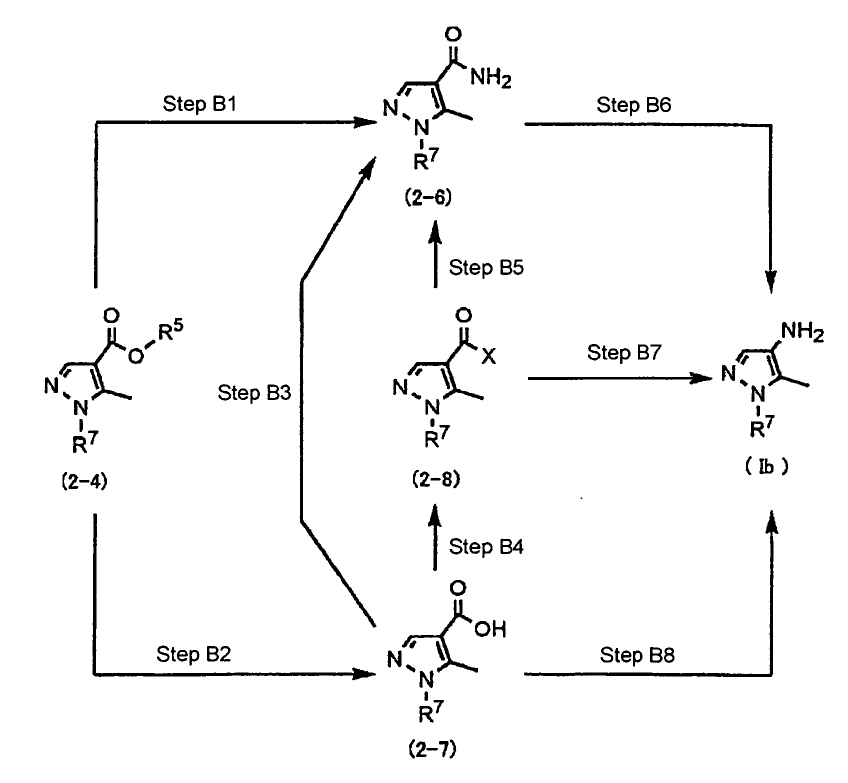

Step B1 is to prepare the compounds represented by the formula (2-6) (in which R⁷ has the same meaning as defined above) by replacing the alkoxy group of the compounds represented by the formula (2-4) with an amino group, and can be carried out, for example, according to methods described in Tetrahedron, vol. 31, 2659 (1975), Can. J. Chem., vol. 47, 3671 (1969), J. Chem. Soc., C, 1969, 1729 and J. Am. Chem. Soc., vol. 82, 2725 (1960).

The reaction for replacing the alkoxy group of the ester with an amino group is usually carried out using an aminating agent such as ammonia gas, aqueous ammonia or a metal amide. The metal amide is not particularly limited so long as it is usually available and such the metal amide includes, for example, sodium amide or potassium amide, and preferably sodium amide.

An amount of the aminating agent used is not particularly limited so long as it is 1 equivalent or more relative to an amount of the compounds of the formula (2-4) used and is usually from 1 to 100 equivalents, preferably from 2 to 50 equivalents, and more preferably from 3 to 40 equivalents.

The reaction is usually carried out in a solvent. The solvent is not particularly limited so long as it does not inhibit the reaction and in a case where the metal amide is used, a protolytic solvent is avoided. In a case where ammonia gas or aqueous ammonia is used as the aminating agent, such the solvent includes, for example, water; alcohols such as methanol, ethanol, propanol, isopropyl alcohol or butanol; ethers such as diethyl ether, tetrahydrofuran or dioxane; amides such as dimethylformamide, dimethylacetamide, N,N-dimethylimidazolidinone or hexamethylphosphoric triamide; sulfoxides such as dimethyl sulfoxide; or a mixture thereof, preferably the alcohols, the ethers, the amides or the sulfoxides, and more preferably the alcohols and the amides. In a case where the metal amide is used as the aminating agent, such the solvent includes ethers such as diethyl ether, tetrahydrofuran or dioxane; amides such as dimethylformamide, dimethylacetamide, N,N-dimethylimidazolidinone or hexamethylphosphoric triamide; sulfoxides such as dimethyl sulfoxide; or a mixture thereof, preferably the ethers, the amides or the sulfoxides, and more preferably the amides.

The reaction temperature is not limited and the reaction is usually carried out at from 0°C to 200°C, preferably at from room temperature to 200°C, and more preferably at from room temperature to 180°C.

Step B2 is to obtain the carboxylic acids represented by the formula (2-7) (in which R⁷ has the same meaning as defined above) by hydrolyzing the compounds represented by the formula (2-4).

The reaction is carried out by a method known as a hydrolysis reaction of an ester and is usually carried out in the presence of an acid or a base.

In a case where the reaction is carried out in the presence of an acid, the acid used is not particularly limited. Such the acid includes, for example, hydrochloric acid, hydrobromic acid or sulfuric acid, preferably hydrochloric acid or sulfuric acid, and more preferably hydrochloric acid.

In a case where the reaction is carried out in the presence of a base, the base used is not particularly limited. Such the base includes, for example, lithium hydroxide, sodium hydroxide, potassium hydroxide, cesium hydroxide, lithium carbonate, sodium carbonate, potassium carbonate, cesium carbonate, lithium hydrogencarbonate, sodium hydrogencarbonate, potassium hydrogencarbonate or cesium hydrogencarbonate, preferably lithium hydroxide, sodium hydroxide, potassium hydroxide, cesium hydroxide, sodium carbonate, potassium carbonate or cesium carbonate, and more preferably sodium hydroxide, potassium hydroxide, cesium hydroxide, sodium carbonate, potassium carbonate or cesium carbonate.

An amount of the acid or the base used is not particularly limited so long as it is 1 equivalent or more relative to the compounds of the formula (2-4) used and is usually from 2 to 20 equivalents, preferably from 2 to 10 equivalents, and more preferably from 2 to 6 equivalents.

The reaction is usually carried out in a solvent. The solvent used is not particularly limited so long as it does not inhibit the reaction. Such the solvent includes, for example, water; alcohols such as methanol, ethanol, propanol, isopropyl alcohol or butanol; ethers such as diethyl ether, tetrahydrofuran or dioxane; amides such as dimethylformamide, dimethylacetamide, N,N-dimethylimidazolidinone or hexamethylphosphoric triamide; sulfoxides such as dimethyl sulfoxide; or a mixture thereof, preferably water, the alcohols, the ethers, the amides or the sulfoxides, and more preferably water or the alcohols.

The reaction temperature is not particularly limited and the reaction is usually carried out at from -20°C to 180°C, preferably at from -20°C to 100°C, and more preferably -10°C to 80°C.

Step B3 is to prepare the carboxylic acid amides represented by the formula (2-6) from the carboxylic acids represented by the formula (2-7) (in which R7 has the same meaning as defined above) and can be carried out, for example, according to methods described in Tetrahedron, vol. 31, 2659 (1975), J. Org. Chem., vol. 24, 1632 (1959), Helv. Chim. Acta., vol. 29, 1438 (1946) or Tetrahedron Lett., vol. 41, 5229 (2000).

The reaction for preparing the carboxylic acid amides from the carboxylic acids is usually carried out by reacting with ammonia gas or aqueous ammonia and is optionally carried out using a dehydrating agent.

An amount of ammonia gas or aqueous ammonia used is not particularly limited so long as it is 1 equivalent or more relative to an amount of the compounds of the formula (2-7) used and is usually from 1 to 100 equivalents, preferably from 2 to 50 equivalents, and more preferably from 3 to 40 equivalents.

In a case where the dehydrating agent is used, the dehydrating agent is not particularly limited and, for example, an ion exchange resin or molecular sieve is used.

The reaction is usually carried out in a solvent. The solvent is not particularly limited so long as it does not inhibit the reaction. Such the solvent includes, for example, water; alcohols such as methanol, ethanol, propanol, isopropyl alcohol or butanol; ethers such as diethyl ether, tetrahydrofuran or dioxane; amides such as dimethylformamide, dimethylacetamide, N,N-dimethylimidazolidinone or hexamethylphosphoric triamide; sulfoxides such as dimethyl sulfoxide; or a mixture thereof, preferably the alcohols, the ethers, the amides or the sulfoxides, and more preferably the alcohols and the amides.

The reaction temperature is not limited and the reaction is usually carried out at from 0°C to 200°C, preferably at from room temperature to 200°C, and more preferably at from room temperature to 180°C.

Step B4 is to obtain the compounds represented by the formula (2-8) (in which X represents a chlorine atom, a bromine atom, a methoxycarbonyloxy group, an ethoxycarbonyloxy group or a phenoxycarbonyloxy group) from the carboxylic acids represented by the formula (2-7) .

In a case where X is a chlorine atom or bromine atom, the reaction is carried out by a method usually known as a synthetic method of an acid chloride or acid bromide using a chlorinating agent or a brominating agent such as oxalyl chloride, thionyl chloride, phosphorus oxychloride, phosphorus pentachloride, oxalyl bromide, thionyl bromide, phosphorus oxybromide or phosphorus pentabromide.

An amount of the chlorinating agent or the brominating agent is not particularly limited so long as it is 1 equivalent or more relative to the compounds represented by the formula (2-7) used and is usually from 1 to 10 equivalents, preferably from 1 to 5 equivalents, and more preferably from 1 to 2 equivalents.

When a base is optionally added, the reaction sometimes quickly progresses. The base is not particularly limited and includes, for example, ammonia; amines such as methylamine, dimethylamine, trimethylamine, ethylamine, diethylamine, triethylamine or diisopropylethylamine; nitrogen-containing heterocyclic compounds such as pyrrole, pyrazole, imidazole, 1,2,3-triazole, 1,2,4-triazole, pyridine, pyrimidine, pyrazine or triazine; or phosphines such as trimethylphosphine, triethylphosphine or triphenylphosphine, and preferably trimethylamine, triethylamine or pyridine.

The reaction is carried out in a solvent or in no solvent. In a case where the solvent is used, the solvent is not particularly limited so long as it does not inhibit the reaction. Such the solvent includes, for example, hydrocarbons such as pentane, hexane, octane, cyclohexane, benzene, toluene or xylene; ethers such as diethyl ether, tetrahydrofuran or dioxane; halogenated solvents such as dichloromethane, chloroform, 1,2-dichloroethane or 1,1,2-trichloroethane; amides such as dimethylformamide, dimethylacetamide, N,N-dimethylimidazolidinone or hexamethylphosphoric triamide; sulfoxides such as dimethyl sulfoxide; or a mixture thereof, preferably the hydrocarbons, the ethers or the halogenated solvents, and more preferably the ethers or the halogenated solvents.

The reaction temperature is not particularly limited and the reaction is usually carried out at from -20°C to 180°C, preferably at from -20°C to 100°C, and more preferably at from -10°C to 80°C.

In a case where X is the methoxycarbonyloxy group, ethoxycarbonyloxy group or phenoxycarbonyloxy group, the reaction is carried out according to a method usually known as a synthetic method of a mixed acid anhydride in the presence of a base using chlorocarbonates such as methyl chlorocarbonate, ethyl chlorocarbonate or phenyl chlorocarbonate.

An amount of the chlorocarbonate used is usually from 1 to 2 equivalents relative to an amount of the compounds of the formula (2-7) used, preferably from 1 to 1.5 equivalents, and more preferably from 1 to 1.2 equivalents.

As the base used, trialkylamine is usually used, and preferably trimethylamine, triethylamine, tripropylamine or diisopropylethylamine is used.

An amount of the base used is not particularly limited so long as it is 1 equivalent or more relative to an amount of the compounds of the formula (2-7) used and is preferably from 1 to 3 equivalents, and more preferably from 1 to 2 equivalents.

The reaction is usually carried out in a solvent. The solvent is not particularly limited so long as it does not inhibit the reaction. Such the solvent includes, for example, hydrocarbons such as pentane, hexane, octane, cyclohexane, benzene, toluene or xylene; ethers such as diethyl ether, tetrahydrofuran or dioxane; halogenated solvents such as dichloromethane, chloroform, 1,2-dichloroethane or 1,1,2-trichloroethane; amides such as dimethylformamide, dimethylacetamide, N,N-dimethylimidazolidinone or hexamethylphosphoric triamide; sulfoxides such as dimethyl sulfoxide; or a mixture thereof, preferably the ethers or the halogenated solvents, and more preferably the ethers.

The reaction temperature is not particularly limited and the reaction is usually carried out at from -100°C to 50°C, preferably at from -70°c to room temperature, and more preferably at from -40°C to room temperature.

Step B5 is to prepare the acid amide derivatives (2-6) from the compounds represented by the formula (2-8) and is carried out by reacting the compounds represented by the formula (2-8) with ammonia gas or aqueous ammonia.

An amount of ammonia gas or aqueous ammonia used is not particularly limited so long as it is 1 equivalent or more relative to an amount of the compounds of the formula (2-8) used and is usually from 2 to 10 equivalents, preferably from 2 to 5 equivalents, and more preferably from 2 to 3 equivalents.

The reaction is usually carried out in no solvent or in a solvent. In a case where the reaction is carried out in the solvent, the solvent is not particularly limited so long as it does not inhibit the reaction, and includes water; alcohols such as methanol, ethanol, propanol, isopropyl alcohol or butanol; hydrocarbons such as pentane, hexane, octane, cyclohexane, benzene, toluene or xylene; ethers such as diethyl ether, tetrahydrofuran or dioxane; amides such as dimethylformamide, dimethylacetamide, N,N-dimethylimidazolidinone or hexamethylphosphoric triamide; sulfoxides such as dimethyl sulfoxide; or a mixture thereof, preferably the ethers, the amides or the sulfoxides, and more preferably the alcohols and the amide. In a case where the metal amide is used as the aminating agent, such the solvent includes ethers such as diethyl ether, tetrahydrofuran or dioxane; halogenated solvents such as dichloromethane, chloroform, 1,2-dichloroethane or 1,1,2-trichloroethane; amides such as dimethylformamide, dimethylacetamide, N,N-dimethylimidazolidinone or hexamethylphosphoric triamide; sulfoxides such as dimethyl sulfoxide; or a mixture thereof, preferably the hydrocarbons, the ethers or the halogenated solvents, and more preferably the ethers.

The reaction temperature is not limited and the reaction is usually carried out at from -20°C to 100°C, preferably at from -20°C to 50°c, and more preferably -20°C to room temperature.

Step B6 is to prepare the 4-aminopyrazoles represented by the formula (Ib) from the acid amides represented by the formula (2-6) by a rearrangement reaction.

This step includes a reaction generally known as Hofmann rearrangement and is carried out, for example, according to methods described in edited by Nippon Kagakukai, Jikken Kagaku Koza fourth edition, vol. 20, 304 (Maruzen, Tokyo) and cited here.

The reaction is usually carried out using a base and hypochlorous acid, chlorous acid, hypobromous acid, bromous acid or the like.

The base used is not particularly limited so long as it is usually an inorganic base. Such the base preferably includes, for example, sodium carbonate, potassium carbonate, sodium hydroxide or potassium hydroxide, and more preferably sodium hydroxide or potassium hydroxide.

An amount of the base used is not particularly limited so long as it is 1 equivalent or more relative to the compounds of the formula (2-6) used and is preferably from 1 to 50 equivalents, and more preferably from 3 to 20 equivalents.

Hypochlorous acid, chlorous acid, hypobromous acid, bromous acid or the like used may be used as such or as a salt thereof in a reaction solution, or the product in the reaction system prepared by blowing chlorine or bromine to an aqueous solution of the above base may be used as such.

An amount of hypochlorous acid, chlorous acid, hypobromous acid, bromous acid or the like used is not particularly limited so long as it is 1 equivalent or more relative to the compounds of the formula (2-6) used and is preferably from 1 to 20 equivalents, and more preferably from 1 to 10 equivalents.

The reaction is usually carried out in a solvent. The solvent is not particularly limited so long as it does not inhibit the reaction, and preferably includes water; lower alcohols such as methanol, ethanol or propanol; or a mixture thereof.

The reaction temperature is not particularly limited and the reaction is usually carried out at from -20°C to 150°C, preferably at from -20°C to 80°C, and more preferably -10°C to 60°C.

Since they sometimes stably exists in the form of the isocyanates or carbamic acids as intermediates after completion of the rearrangement reaction, a hydrolysis treatment is sometimes further required.

The hydrolysis treatment is usually carried out using a base or an acid. As a base used, the base usually used in the present reaction is used. An amount of the base used is not particularly limited so long as it is 1 equivalent or more and is usually from 1 to 50 equivalents, preferably from 1 to 20 equivalents, and more preferably from 1 to 10 equivalents. The temperature of hydrolysis is not particularly limited and the hydrolysis is usually carried out at from 0°C to 120°C, preferably at from 0°C to 80°C, and more preferably at from room temperature to 80°C.

Step B7 is to prepare the 4-amino-5-methylpyrazoles represented by the formula (Ib) from the compounds represented by the formula (2-8) through the acid azide compounds or the hydroxamic acids by a rearrangement reaction.

In a case where it is through the acid azide compounds, this step includes a reaction generally known as Curtius rearrangement and in a case where it is through the hydroxamic acids, the step includes the reaction generally known as Lossen rearrangement. These procedures of the reaction are carried out, for example, according to methods described in edited by Nippon kagakukai, Jikken Kagaku Koza fourth edition, vol. 20, 305 (Maruzen, Tokyo), edited by Nippon Kagakukai, Jikken Kagaku Koza fourth edition, vol. 20, 306 (Maruzen, Tokyo) or cited here.

In a case of Curtius rearrangement, since the reaction is through the acid azides, reacting the compounds represented by the formula (2-8) with a metal azide, and subsequently carrying out the rearrangement reaction and hydrolysis to obtain the desired 4-aminopyrazoles represented by the formula (Ib).

The metal azide used is not particularly limited and sodium azide or potassium azide is usually used.

An amount of the metal azide used is not particularly limited so long as it is usually 1 equivalent or more relative to the compounds represented by the formula (2-8) used and is preferably from 1 to 10 equivalents, and more preferably from 1 to 5 equivalents.

The reaction is usually carried out in a solvent. The solvent used is not particularly limited so long as it does not inhibit the reaction. Such the solvent includes, for example, hydrocarbons such as hexane, octane, benzene, toluene or xylene; halogenated solvents such as dichloromethane, chloroform, 1,2-dichloroethane or 1,1,2-trichloroethane; protolytic solvents such as water, methanol, ethanol, propanol or t-butyl alcohol; ethers such as diethyl ether, tetrahydrofuran or dioxane; or a mixture thereof, preferably the hydrocarbons or the protolytic solvents, and more preferably benzene, toluene, methanol, ethanol and t-butyl alcohol.

The reaction temperature is not particularly limited and the reaction is usually carried out at from room temperature to 180°C, preferably at from room temperature to 150°C, and more preferably at from room temperature to 120°C.

In a case of Lossen rearrangement, since the reaction is through the hydroxamic acids, reacting the compounds represented by the formula (2-8) with usually hydroxylamine or its salt, or nitromethane and then carrying out the rearrangement reaction to obtain the 4-amino-5-methylpyrazole derivatives represented by the formula (Ib).

An amount of hydroxylamine or its salt, or nitromethane used is not particularly limited so long as it is 1 equivalent or more relative to the compounds represented by the formula (2-8) used and is preferably from 1 to 10 equivalents, and more preferably from 1 to 5 equivalents.

The reaction is usually carried out in a solvent. The solvent used is not particularly limited so long as it does not inhibit the reaction and the reaction is usually carried out in water under co-existence of an acid.

As the co-existed acid, an inorganic acid is usually used and preferably sulfuric acid or phosphoric acid is used.

The reaction temperature is not particularly limited and the reaction is usually carried out at from room temperature to 200°c, preferably at from room temperature to 180°C, and more preferably at from room temperature to 160°C.

Step B8 is to prepare the 4-aminopyrazoles represented by the formula (Ib) from the carboxylic acids represented by the formula (2-7) by a rearrangement reaction and the reaction is usually known as Schmidt rearrangement reaction. These procedures of reaction are carried out, for example, according to the methods described in edited by Nippon Kagakukai, Jikken Kagaku Koza fourth edition, vol. 20, 304 (Maruzen, Tokyo) or cited here.

The reaction is usually carried out by reacting the compounds represented by the formula (2-7) with hydrazoic acid under co-existence of an acid.

As hydrazoic acid, hydrazoic acid is usually used as such or the compound prepared in the reaction system by adding a metal azide into an acid is used.

An amount of hydrazoic acid used is not particularly limited so long as it is 1 equivalent or more relative to the compounds represented by the formula (2-7) used and is preferably from 1 to 10 equivalents, and more preferably from 1 to 3 equivalents.

The co-existed acid is not particularly limited. As such the acid, a strong acid is usually used, and preferably polyphosphoric acid, sulfuric acid or trifluoroacetic acid is used and more preferably polyphosphoric acid or sulfuric acid is used.

The reaction is usually carried out in a solvent. The solvent used is not particularly limited so long as it does not inhibit the reaction and such the solvent includes, for example, hydrocarbons such as hexane, octane, benzene, toluene or xylene; halogenated solvents such as dichloromethane, chloroform, 1,2-dichloroethane or 1,1,2-trichloroethane; protolytic solvents such as water, methanol, ethanol, propanol or t-butyl alcohol; or a mixture thereof, preferably the hydrocarbons or the protolytic solvents, and more preferably benzene, toluene, water, methanol and ethanol.

The reaction temperature is not particularly limited and the reaction is usually carried out at from room temperature to 180°C, preferably at from room temperature to 150°C, and more preferably at from room temperature to 120°C.

Step C in the acetoacetic acid ester method of the present invention is to prepare the compounds represented by the formula (2-5) by hydrogenating the compounds represented by the formula (2-1) and the reaction is carried out by usual catalytic hydrogenation.

### Step C:

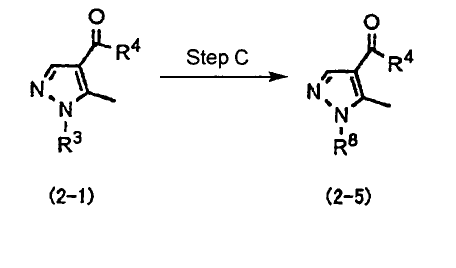

The catalyst used in the present step is not particularly limited so long as it is usually used for catalytic hydrogenation. Such the catalyst includes, for example, a palladium-carbon catalyst, platinum-carbon catalyst, Raney nickel or Wilkinson complex, preferably the palladium-carbon catalyst or Raney nickel, and more preferably the palladium-carbon catalyst.

The pressure of hydrogen in the present step is not particularly limited so long as it is 1 atm or more and is usually from 1 to 10 atm, preferably from 1 to 5 atm, and more preferably from 1 to 3 atm.

The present step (reaction) is usually carried out in a solvent. The solvent is not particularly limited so long as it is inactive for the present reaction and such the solvent includes water; alcohols such as methanol, ethanol, propanol or isopropyl alcohol; or esters such as methyl acetate, ethyl acetate and butyl acetate, preferably water, methanol or ethyl acetate, and more preferably methanol.

The reaction temperature of the present step is not particularly limited but the reaction is usually carried out at from room temperature to 150°C, and preferably at room temperature.

After completion of the reactions of respective steps of Step A, Step B and Step C, the mixture is made acidic, neutral or basic corresponding to a physical property of the products after post-treatment, and isolation operation is carried out. After the isolation, the products can be used in the subsequent step as such or, if necessary, with conventional purifications such as distillation, recrystalization or chromatography.

### (3) Acetylacetone method

The acetylacetone method of the present invention includes Step A and Step B (Steps B1, B2-1 and B2-2) described hereinafter. In this method, the compounds represented by the following formulae (3-1), (3-2), (3-3) and (3-4) are prepared as synthetic intermediates and the final products are the compounds represented by the formula (Ic). 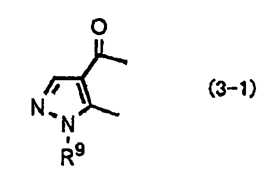 (wherein R⁹ represents a straight or branched alkyl group having 3 or more of carbon chains which may have optionally a substituent(s), a cycloalkylmethyl group which may have optionally a substituent(s), a straight or branched alkenyl group which is the alkenyl group bonded at the sp³ carbon atom and may have optionally a substituent(s), a straight or branched alkynyl group which is the alkynyl group bonded at the sp³ carbon atom and may have optionally a substituent(s) or a cycloalkenylmethyl group); 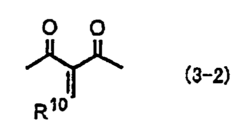 (wherein R¹⁰ represents a lower alkoxy group, a phenoxy group or a dialkylamino group); 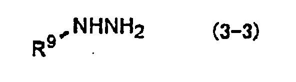 (wherein R⁹ has the same meaning as defined above); 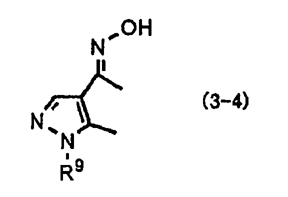 (wherein R⁹ has the same meaning as defined above); and 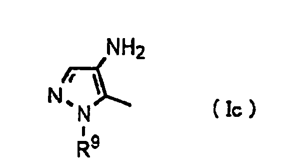 (wherein R⁹ has the same meaning as defined above).

When R⁹ represents "a straight or branched alkyl group having 3 or more of carbon chains which may have optionally a substituent(s), it is a C₃-C₆ alkyl group. Such the alkyl group represents a propyl group, isopropyl group, butyl group, isobutyl group, s-butyl group, t-butyl group, pentyl group, isopentyl group, s-pentyl group, t-pentyl group, neopentyl group and hexyl group, and preferably the methyl group, ethyl group, propyl group, isopropyl group, butyl group, isobutyl group, s-butyl group, pentyl group, isopentyl group, s-pentyl group, neopentyl group and hexyl group.

When R⁹ represents "an alkyl group which may have optionally a substituent(s)", "the substituent" represents a phenyl group.

When R⁹ represents "a cycloalkylmethyl group which may have optionally a substituent(s)", "the cycloalkylmethyl group" is a cyclopropylmethyl group, cyclobutylmethyl group, cyclopentylmethyl group or cyclohexylmethyl group, and preferably the cyclopropylmethyl group, cyclobutylmethyl group or cyclopentylmethyl group.

When R⁹ represents "a cycloalkylmethyl group which may have optionally a substituent(s)", "the substituent" represents a lower alkyl group or phenyl group. The lower alkyl includes, for example, a methyl group, ethyl group, propyl group, isopropyl group, butyl group and t-butyl group. The preferable substituent includes the methyl group, ethyl group or phenyl group, and more preferably the methyl group or phenyl group.

When R⁹ represents "a straight or branched alkenyl group which is the alkenyl group bonded at the sp³ carbon atom and may have optionally a substituent(s)", "the alkenyl group" is a C₃-C₈ alkenyl group. Such the alkenyl group is a 2-propenyl group, 1-methyl-2-propenyl group, 2-methyl-2-propenyl(metallyl) group, 2-ethyl-2-propenyl group, 2-butenyl group, 1-methyl-2-butenyl group, 2-methyl-2-butenyl group, 1-ethyl-2-butenyl group, 3-butenyl group, 1-methyl-3-butenyl group, 2-methyl-3-butenyl group, 1-ethyl-3-butenyl group, 2-pentenyl group, 1-methyl-2-pentenyl group, 2-methyl-2-pentenyl group, 3-pentenyl group, 1-methyl-3-pentenyl group, 2-methyl-3-pentenyl group, 4-pentenyl group, 1-methyl-4-pentenyl group, 2-methyl-4-pentenyl group, 5-hexenyl group, 6-heptenyl group and 7-octenyl group, preferably the 2-propenyl, 2-butenyl group, 2-methyl-2-propenyl(metallyl) group, 2-methyl-2-butenyl group, 2-pentenyl group, 3-pentenyl group and 4-pentenyl group, and more preferably the 2-propenyl, 2-butenyl group, 2-methyl-2-propenyl(metallyl) group and 2-methyl-2-butenyl group.

When R⁹ represents "a straight or branched alkenyl group which is the alkenyl group bonded at the sp³ carbon atom and may have optionally a substituent(s)", "the substituent" represents a phenyl group.

When R⁹ represents "a straight or branched alkynyl group which is the alkynyl group bonded at the sp³ carbon atom and may have optionally a substituent(s)", "the alkynyl group" is a C₃-C₈ alkynyl group. Such the alkynyl group includes a 2-propynyl group, 1-methyl-2-propynyl group, 2-butynyl group, 1-methyl-2-butynyl group, 1-ethyl-2-butynyl group, 3-butynyl group, 1-methyl-3-butynyl group, 2-methyl-3-butynyl group, 1-ethyl-3-butynyl group, 2-pentynyl group, 1-methyl-2-pentynyl group, 3-pentynyl group, 1-methyl-3-pentynyl group, 2-methyl-3-pentynyl group, 4-pentynyl group, 1-methyl-4-pentynyl group, 2-methyl-4-pentynyl group, 5-hexynyl group, 6-heptynyl group and 7-octynyl group, preferably the 2-propynyl group, 2-butynyl group, 3-butynyl group, 2-pentynyl group, 3-pentynyl group and 4-pentynyl group, and more preferably the 2-propynyl group, 2-butynyl group and 3-butynyl group.

When R⁹ represents "a straight or branched alkynyl group which is the alkynyl group bonded at the sp³ carbon atom and may have optionally a substituent(s)", "the substituent" represents a phenyl group.

When R⁹ represents "a cycloalkenylmethyl group", "the cycloalkenyl group" represents a cyclopentenyl group or cyclohexenyl group.

When R¹⁰ represents "a lower alkoxy group", "the lower alkoxy group" is a straight or branched C₁-C₄ alkoxy group. Such the alkoxy group includes, for example, a methoxy group, ethoxy group, propoxy group, isopropoxy group, butoxy group, isobutoxy group or t-butoxy group, preferably the methoxy group, ethoxy group or t-butoxy group, and more preferably the methoxy group or ethoxy group.

When R¹⁰ represents "a dialkylamino group", "the dialkylamino group" includes, for example, a dimethylamino group, diethylamino group, dipropylamino group and ethylmethylamino group, and preferably the dimethylamino group.

Step A of the acetylacetone method of the present invention is to prepare the compounds represented by the formula (3-1) regioselectively by reacting the compounds represented by the formula (3-2) with the compounds represented by the formula (3-3) under co-existence of an acid.

### Step A:

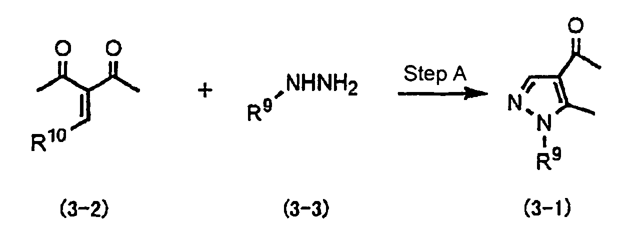

The acid used in the present step is not particularly limited so long as it is an usual Bronsted acid. Such the acid includes, for example, hydrogen chloride, hydrochloric acid, hydrobromic acid, hydroiodic acid, sulfuric acid, nitric acid, methanesulfonic acid, p-toluenesulfonic acid, formic acid, acetic acid or a mixture thereof, preferably hydrogen chloride, hydrochloric acid, sulfuric acid, methanesulfonic acid or p-toluenesulfonic acid, and more preferably hydrogen chloride, hydrochloric acid or sulfuric acid.

An amount of the acid used is not particularly limited so long as it is 1 equivalent or more relative to the compounds of the above formula (3-3) and is usually from 1 to 10 equivalents, preferably from 1 to 4 equivalents, and more preferably from 1 to 1.5 equivalents.

The acid used may be added separately from the compounds of the above formula (3-3) and may be used as a salt of the compounds of the formula (3-3).

The reaction is usually carried out in a solvent. The solvent is not particularly limited so long as it does not inhibit the reaction. Such the solvent includes, for example, water; alcohols such as methanol, ethanol, propanol, isopropyl alcohol or butanol; ethers such as diethyl ether or tetrahydrofuran; or halogenated solvents such as dichloromethane, chloroform or 1,2-dichloroethane, preferably the alcohols, and more preferably methanol or ethanol.

The reaction temperature is not particularly limited and the reaction is usually carried out at from -20°C to 50°C, and preferably at from -5°C to room temperature.

Step B of the acetylacetone method of the present invention is to prepare the 4-amino-5-methylpyrazole derivatives represented by the formula (Ic) by converting the compounds represented by the formula (3-1) obtained in step A to the compounds (the hydroxyimino derivatives) represented by the formula (3-4) and carrying out Beckman rearrangement reaction.

### Step B:

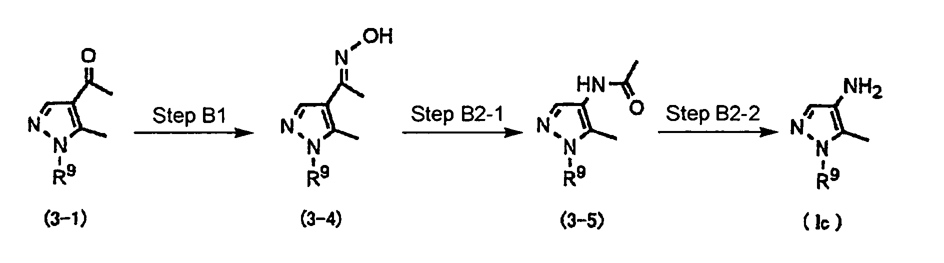

Step B1 is to prepare the compounds represented by the formula (3-4) (wherein R⁹ has the same meaning as defined above) by hydroxyiminating the carbonyl group of the compounds represented by the formula (3-1) and can be carried out, for example, according to the methods described in J. Am. Chem. Soc., vol. 66, 1293 (1944), J. Org. Chem., vol. 3, 300 (1938) and Chem. Ber., vol. 23, 1452 (1890).

The reaction for hydroxyiminating the carbonyl group is usually carried out using hydroxylamine or its salt in the presence of a base. The salt is not particularly limited so long as it is usually available and such the salt includes, for example, hydroxylamine hydrochloride or hydroxylamine sulfate.

In a case where the reaction is carried out in the presence of a base, the base used is not particularly limited. Such the base includes, for example, inorganic bases such as lithium hydroxide, sodium hydroxide, potassium hydroxide, cesium hydroxide, lithium carbonate, sodium carbonate, potassium carbonate, cesium carbonate, lithium hydrogencarbonate, sodium hydrogencarbonate, potassium hydrogencarbonate, cesium hydrogencarbonate, sodium acetate or potassium acetate, ammonia and amines such as trimethylamine, triethylamine, diisopropylethylamine, pyridine or lutidine, and preferably sodium hydroxide, potassium hydroxide, sodium carbonate, potassium carbonate, sodium hydrogencarbonate, potassium hydrogencarbonate, sodium acetate, potassium acetate, sodium acetate, triethylamine and pyridine.

An amount of the hydroxylamine and the base used is not particularly limited so long as it is 1 equivalent or more relative to the compounds of the formula (3-1) used and is usually form 1 to 10 equivalents, and preferably from 1 to 5 equivalents.

The reaction is usually carried out in a solvent. The solvent is not particularly limited so long as it does not inhibit the reaction and such the solvent includes, for example, water; alcohols such as methanol, ethanol, propanol, isopropyl alcohol or butanol; amides such as dimethylformamide, dimethylacetamide, N,N-dimethylimidazolidinone or hexamethylphosphoric triamide; sulfoxides such as dimethyl sulfoxide; or a mixture thereof, and preferably water, the alcohols or the mixture thereof.

The reaction temperature is not limited and the reaction is usually carried out at from 0°C to 200°C, and preferably at from room temperature to 80°C.

Step B2 is to obtain the 4-amino-5-methylpyrazole derivatives represented by the formula (Ic) (wherein R⁹ has the same meaning as defined above) (Step B2-2) by synthesizing the N-(5-methylpyrazol-4-yl)acetamides represented by the formula (3-5) (wherein R⁹ has the same meaning as defined above) from the compounds represented by the formula (3-4) using a rearrangement reaction (Step B2-1) and hydrolyzing the acetamides simultaneously with the post-treatment of the reaction.

Step B2-1 includes a reaction generally known as Beckman rearrangement reaction and the reaction is carried out, for example, according to the methods described in edited by Nippon Kagakukai, Jikken Kagaku Koza fourth edition, vol. 20, 308 (Maruzen, Tokyo) and cited here.

The reaction is usually carried out using a strong acid such as phosphorous pentachloride, phosphorous oxychloride, diphosphorous pentaoxide, polyphosphoric acid, concentrated sulfuric acid and trifluoroacetic acid, or sulfonyl chloride-pyridine. Preferably the strong acid such as polyphosphoric acid and concentrated sulfuric acid is used.

An amount of the strong acid used is not particularly limited so long as it is 1 equivalent or more relative to the compounds of the formula (3-4) and is preferably from 1 to 20 equivalents, and more preferably from 1 to 10 equivalents.

The reaction is usually carried out in no solvent and the solvent is sometimes utilized so long as it does not inhibit the reaction. Such the solvent includes, for example, amides such as dimethylformamide, dimethylacetamide, N,N-dimethylimidazolidinone or hexamethylphosphoric triamide; and halo-alkanes such as methylene chloride, chloroform and 1,2-dichloroethane.

The reaction temperature is not particularly limited and the reaction is usually carried out at from -20°C to 200°C, and preferably at from 0°C to 180°C.

Step B2-2 is a hydrolysis step and is accomplished by diluting the reaction mixture with water and (over)heating it.

An amount of diluting water is not particularly limited and is usually from 1 to 10 times of the acid used, and preferably from 1 to 5 times. The temperature of the hydrolysis is not particularly limited and the hydrolysis is usually carried out at from room temperature to 150°C, and preferably at from 80°C to 120°C.

Further, the hydrolysis step can be also carried out under a basic condition.

As the base used, a strong base such as sodium hydroxide and potassium hydroxide is used.

An amount of the base used is not particularly limited so long as it is 1 equivalent or more and is usually from 1 to 50 equivalents, and preferably from 1 to 20 equivalents.

The reaction is usually carried out in water and the solvent is sometimes utilized so long as it does not inhibit the reaction. As such the solvent, for example, alcohols such as methanol, ethanol, propanol, butanol and ethylene glycol are used.
The temperature of hydrolysis is not particularly limited and the hydrolysis is usually carried out at from room temperature to 200°C, and preferably at from 80°C to 180°C.

After completion of the reactions of Step A and Step B, the mixture is made acidic, neutral or basic corresponding to physical property of the products after post-treatment, and isolation operation is carried out. After the isolation, the products can be used in the subsequent step as such or, if necessary, with conventional purifications such as distillation, recrystalization or chromatography.

Further, Step B1 of the acetylacetone method of the present invention includes the step for preparing the compounds represented by the formula (3-1") by hydrogenation from the compounds represented by the following formula (3-1'), and the hydrogenation is carried out by usual catalytic hydrogenation and can be appropriately carried out according to the method of the hydrogenation in Step C of the above acetoacetic acid ester method.

Namely, the present invention provides a method for preparing the 4-amino-5-methylpyrazole derivatives represented by the formula (Ic'): 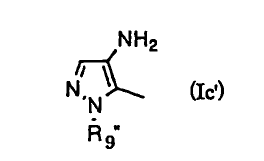 (wherein R₉" represents a straight or branched alkyl group having 3 or more of carbon chains which may have optionally a substituent(s) or a cycloalkylmethyl group which may have optionally a substituent(s)),
comprising preparing the compounds represented by the following formula (3-1"): 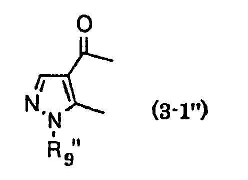 (wherein R₉" has the same meaning as defined above) by hydrogenating the compounds represented by the following formula (3-1'): 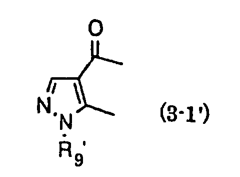 (wherein R₉' represents a straight or branched alkenyl group which is the alkenyl group bonded at the sp³ carbon atom and may have optionally a substituent(s), a straight or branched alkynyl group which is the alkynyl group bonded at the sp³ carbon atom and may have optionally a substituent(s) or a cycloalkenylmethyl group); hydroxyiminating the compounds (3-1") to obtain the compounds represented by the following formula (3-4'): 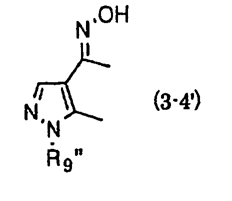 (wherein R₉" has the same meaning as defined above); and further carrying out Beckman rearrangement reaction.

In the following, Examples and Comparative Examples of the present invention are shown and the present invention is explained in more detail.

### Example

### Example 1 Lithiation method

### Example 1-1

### 1-Isobutylpyrazole (Step A)

Isobutyl bromide (176 ml, 1.62 mol), tetra-n-butylammonium bromide (4.8 g, 0.015 mol) and 40% aqueous sodium hydroxide solution (192 ml, 2.94 mol) were added to a solution of 1H-pyrazole (100.0 g, 1.47 mol) in methylcyclohexane (200 ml) and the mixture was refluxed for 5.5 hours while violently stirring at an oil bath temperature of 110°C. After the mixture was cooled to room temperature, the organic phase was separated and washed with water and then brine. The thus obtained organic phase was dried over anhydrous magnesium sulfate and concentrated to obtain the desired compound (183.1 g, 1.47 mol) containing approximately 0.5% of methylcyclohexane. Yield: 100%
Mass (EI); M/z: 124 (M⁺), 109, 81.
NMR (200 MHz, CDCl₃); δ (ppm): 0.90 (6H, d, J=6.9 Hz), 2.21 (1H, brhep, J=6.9 Hz), 3.92 (2H, d, J=7.3 Hz), 6.23 (1H, dd, J=1.7 Hz, J=2.1 Hz). 7.35 (1H, d, J=2.1 Hz), 7.50 (1H, d, J=1.7 Hz).

### Example 1-2

### 1-Cyclobutylmethylpyrazole (Step A)

Cyclobutylmethyl bromide (45 ml, 0.40 mol), tetra-n-butylammonium bromide (1.2 g, 0.004 mol) and 40% aqueous sodium hydroxide solution (48 ml, 0.734 mol) were added to a solution of 1H-pyrazole (25.0 g, 0.37 mol) in methylcyclohexane (50 ml) and the mixture was refluxed for 5.5 hours while vigorously stirring at an oil bath temperature of 110°C. After the mixture was cooled to room temperature, the organic phase is separated and washed with water and then brine. The thus obtained organic phase was dried over anhydrous magnesium sulfate and concentrated to obtain the desired compound (52.1 g, 0.360 mol) containing approximately 0.6% of methylcyclohexane. Yield: 98%
Mass (EI); M/z: 136 (M⁺), 107, 81, 64.
NMR (200 MHz, CDCl₃); δ (ppm): 1.69-2.15 (6H, m), 2.82 (1H, hep, J=7.4 Hz), 4.14 (2H, d, J=7.4 Hz), 6.22 (1H, dd, J=2.0 Hz, J=2.2 Hz), 7.34 (1H, d, J=2.2 Hz), 7.49 (1H, d, J=2.0 Hz).

### Example 1-3

### 1-Neopentylpyrazole (Step A)

Neopentyl bromide (45 ml, 0.324 mol), tetra-n-butylammonium bromide (0.95 g, 0.003 mol) and 40% aqueous sodium hydroxide solution (38 ml, 0.581 mol) were added to a solution of 1H-pyrazole (20.0 g, 0.294 mol) in methylcyclohexane (40 ml) and the mixture was stirred under reflux for 4 hours while vigorously stirring at an oil bath temperature of 130°C. Further, tetra-n-butylammonium bromide (8.55 g, 0.03 mol) was added thereto, the mixture was stirred under reflux for 5 hours and the reaction mixture was left to stand for 12 hours at room temperature. (Since the reaction was not totally progressed), 1,2,4-trimethylcyclohexane (40 ml) was added thereto, the mixture was stirred under reflux at an oil bath temperature of 160°C for 5 hours and the reaction mixture was left to stand for 12 hours at room temperature. Further, neopentyl bromide (8.1 ml, 0.06 mol) was added thereto and the mixture was stirred under reflux at an oil bath temperature of 160°C for 4 days. After the reaction mixture was cooled to room temperature, water was added thereto and the mixture was extracted with methylcyclohexane. After the extract layer was washed with water and then brine, it was dried over anhydrous magnesium sulfate and concentrated. The thus obtained oil (19.44 g) was distilled under reduced pressure (boiling point: 42-110°C) to obtain a mixture of the desired compound, 1-butylpyrazole, 1,2,4-trimethylcyclohexane and methylcyclohexane of approximately 4:3:3:1.
Mass (EI); M/z: 138 (M⁺).
NMR (200 MHz, CDCl₃); δ (ppm): 0.96 (9H, s), 3.92 (2H, s), 6.24 (1H, dd, J=1.7 Hz, J=2.1 Hz), 7.34 (1H, d, J=2.1 Hz), 7.49 (1H, d, J=1.7 Hz).

### Comparative Example 1-1

### 1-Isobutylpyrazole (the method described Synthetic Commun., vol. 20, 2849 (1990))

1H-pyrazole (5.1 g, 74.91 mmol), potassium hydroxide (9.1 g, 162.18 mmol) and tetrabutylammonium bromide (2.0 g, 6.20 mmol) were sufficiently mixed in a mortar, and the mixture was transferred to a flask and irradiated with a ultrasonic wave for 50 minutes using a ultrasonic washing machine. Isobutyl iodide (8.8 ml, 76.47 mmol) was added thereto and the mixture was stirred for 60 hours. Water was poured to the reaction mixture and the mixture was extracted with methanol-chloroform (1:10). The extract layer was dried, concentrated and distilled to obtain the desired compound (3.97 g, 30.52 mmol). Yield: 41%

### Example 1-4

### 1-Isobutyl-5-methylpyrazole (Step B)

A mechanical stirrer, Dimroth, a thermometer and a rubber stopper were attached to a 2L 4-neck flask and 1-isobutylpyrazole (104.0 g, 0.838 mol) synthesized in Example 1-1 was placed therein under a nitrogen atmosphere. Further, THF (500 ml) was added thereto to make a solution and the solution was cooled to -50°C. 10N n-Butyl lithium (100 ml, 1.000 mol) was added dropwise thereto over 30 minutes while stirring (so as the temperature was not to exceed -20°C). Thereafter, after the reaction mixture was stirred at from -20 to -15°C for 1.5 hours, the rubber stopper was replaced with a 200ml dropping funnel with pressure equalizing arm and a solution of methyl iodide (63 ml, 1.012 mol) in THF (100 ml) was added dropwise thereto over 40 minutes (so as the temperature was not to exceed -10°C). After the reaction mixture was stirred at from -30 to -15°C for 1.5 hours, water (200 ml) was added dropwise thereto over 20 minutes (so as the temperature was not to -10°C). The reaction mixture was raised to room temperature and concentrated up to approximately 500 ml. Water (500 ml) was added thereto and the mixture was extracted with ethyl acetate-hexane (2:1). The extract phase was washed with water and then brine, dried over anhydrous sodium sulfate and concentrated to obtain the desired compound (107.8 g, 0.780 mol). Yield: 93%
NMR (270 MHz, CDCl₃); δ (ppm): 0.91 (6H, d, J=6.9 Hz), 2.20 (1H, brhep, J=6.9 Hz), 2.27 (3H, s), 3.81 (2H, d, J=6.9 Hz), 5.98 (1H, d, J=1.1), 7.38 (1H, d, J=1.1 Hz).

### Comparative Example 1-2

### 1-Isobutyl-5-methylpyrazole (the method described in Liebigs Ann. Chem., Vol. 625, 55 (1959))

1.59N n-Butyl lithium (2.6 ml, 4.134 mmol) was added dropwise to a solution of 1-isobutylpyrazole (333.7 mg, 2.719 mmol) in ether (5 ml) while stirring at -15°C. After the reaction mixture was stirred at the same temperature for 40 minutes, a solution of dimethyl sulfate (0.4 ml, 4.227 mmol) in ether (3 ml) was added thereto. After the reaction mixture was stirred for 1 hour, water was added thereto and the mixture was extracted with ethyl acetate. The extract phase was dried over anhydrous sodium sulfate and concentrated to obtain a mixture (approximately 420 mg) of the desired compound and the starting material of 3.6:1. Conversion yield: 78%

### Example 1-5

### 1-Isobutyl-5-methyl-4-nitropyrazole (Step C1)

A mechanical stirrer, Dimroth, a thermometer and a 200ml dropping funnel with pressure equalizing arm were attached to a 2L 4-neck flask and 1-isobutyl-5-methylpyrazole (107.8 g, 0.780 mol) synthesized in Example 1-2 was placed therein. After the mixture was cooled to 10°C in an ice-bath, 97% sulfuric acid (350 ml, 6.369 mol) was added dropwise thereto over 40 minutes while stirring. The reaction temperature was raised to 50°C at the maximum. After the mixture was cooled to 10°C, the ice-bath was removed and 60% nitric acid (85 ml, 1.085 mol) was added dropwise thereto over 2.5 hours (so as the reaction temperature was not to exceed 60°C), and the mixture was stirred for further 3 hours. The reaction mixture was cooled to -10°C in a salt-ice bath and 25% aqueous ammonia (950 ml) was added dropwise thereto over 1 hour (so as the reaction temperature was not to exceed 60°C). After the mixture was cooled to room temperature, it was extracted with ethyl acetate. The organic phase was dried over anhydrous sodium sulfate and concentrated to obtain the desired compound (136.0 g, 0.742 mol).
Yield from 1-isobutylpyrazole: 89%
NMR (270 MHz, CDCl₃); δ (ppm): 0.94 (6H, d, J=6.9 Hz), 2.20 (1H, brhep, J=6.9 Hz), 2.27 (3H, s), 3.88 (2H, d, J=6.9 Hz), 8.09 (1H, s).

### Example 1-6

### 4-Amino-1-isobutyl-5-methylpyrazole (Step C2)

A solution of 1-isobutyl-5-methyl-4-nitropyrazole (146 g, 796.9 mmol) in methanol (400 ml) was placed in a 1L autoclave vessel and water-containing 10% palladium-carbon (palladium 4.85%) (8.7 g, 3.97 mmol) was added thereto. After the vessel was closed, degassing and hydrogen replacement (8-15 kg/cm2) were carried out and the mixture was heated and stirred for 30 minutes. The temperature of the mixture was finally raised to 130°C by the heat of the reaction. After the mixture was cooled to 40°C, degassing and nitrogen replacement were repeated three times while stirring. The reaction mixture was filtered using Celite and washed with methanol. The filtrate and the washing liquid were combined and concentrated to obtain the desired compound (117.1 g, 764.20 mmol) as crystals. Yield: 96%
NMR (200 MHz, CDCl₃); δ (ppm): 0.89 (6H, d, J=7.0 Hz), 2.15 (3H, s), 2.15 (1H, brquint, J=7.0 Hz), 2.49 (2H, brs), 2.75 (1H, brquint, J=7.6 Hz), 3.96 (2H, d, J=7.6 Hz), 7.12 (1H, s).

### Example 1-7

### 4-Amino-1-cyclobutylmethyl-5-methylpyrazole (Step B, Step C1 and Step C2)

1.57M Butyl lithium (116.0 ml, 182.12 mmol) was added dropwise to a solution of 1-cyclobutylmethylpyrazole (20.68 g, 151.85 mmol) obtained in Example 1-2 in tetrahydrofuran (120 ml) at -40°C and the mixture was stirred at from -50°C to -20°C for 2 hours. Methyl iodide (11.5 ml, 184.73 mmol) was added dropwise thereto over 10 minutes so as the temperature was to maintain form -30°C to -10°C and the mixture was further stirred for 30 minutes. Water was poured to the reaction mixture and the mixture was extracted with ethyl acetate. The extract layer was dried and concentrated to obtain 1-cyclobutylmethyl-5-methylpyrazole (23.23 g). Concentrated sulfuric acid (84 ml, 1528.6 mmol) was added dropwise to the 1-cyclobutylmethyl-5-methylpyrazole thus obtained over 20 minutes while cooling in an ice-bath and then concentrated nitric acid (18 ml, 229.7 mmol) was added dropwise thereto over 20 minutes. The ice-bath was removed and the mixture was stirred for 2 hours. The mixture was cooled to -20°C, 25% aqueous ammonia (224 ml) was added dropwise thereto over 30 minutes and the mixture was extracted with ethyl acetate. The extract layer was dried and concentrated to obtain 1-cyclobutylmethyl-5-methyl-4-nitropyrazole (30.52 g). The 1-cyclobutylmethyl-5-methyl-4-nitropyrazole thus obtained was transferred to a 1L autoclave vessel and methanol (250 ml) and water-containing 10% palladium-carbon (palladium 4.85%) (1.75 g, 0.798 mmol) were added thereto. After degassing, hydrogen was replaced at 10 atm and the mixture was stirred at 70°C for 5 hours. After the mixture was cooled to 45°C, degassing-nitrogen replacement was repeated three times and the reaction mixture was filtered using Celite. The filtrate was concentrated to obtain the desired compound (24.83 g, 150.27 mmol). Total yield: 99%
Mass (EI); M/z: 165 (M⁺), 150, 137, 110, 97, 83, 70, 56.
NMR (200 MHz, CDCl₃); δ (ppm): 1.70-2.10 (6H, m), 2.15 (3H, s), 2.49 (2H, brs), 2.75 (1H, brquint, J=7.6 Hz), 3.75 (2H, d, J=7.6 Hz), 7.14 (1H, s).

### Example 1-8

### 1-Neopentyl-5-methyl-4-nitropyrazole (Step B and Step C1)

1.57M Butyl lithium (17.0 ml, 26.69 mmol) was added dropwise to a solution of a mixture of 1-neopentylpyrazole (3.03 g) obtained in Example 1-3 in tetrahydrofuran (30 ml) at -15°C and the mixture was stirred at the same temperature for 40 minutes. Methyl iodide (1.70 ml, 27.31 mmol) was added dropwise thereto over 3 minutes and further, the mixture was stirred for 30 minutes. Water was poured to the reaction mixture and the mixture was extracted with ethyl acetate. The extract layer was dried and concentrated. Concentrated sulfuric acid (9 ml, 163.8 mmol) was added dropwise to the concentrated solution over 20 minutes while cooling in an ice-bath and then concentrated nitric acid (3 ml, 38.3 mmol) was added dropwise thereto over 2 minutes. The ice-bath was removed and the mixture was stirred for 2 hours. Further, concentrated sulfuric acid (9 ml, 163.8 mmol) was added dropwise thereto over 5 minutes and then concentrated nitric acid (4 ml, 51.1 mmol) was added dropwise thereto over 3 minutes, and the mixture was stirred for 1 hour. The mixture was cooled in an ice-bath, 25% aqueous ammonia (53 ml) was added dropwise thereto over 15 minutes and the mixture was extracted with ethyl acetate. The extract layer was dried and concentrated, and the residue was purified by silica gel column chromatography (eluent; hexane~ethyl acetate:hexane = 1:10) to obtain the desired compound (2.835 g, 14.37 mmol). Yield from 1H-pyrazole: 65%
NMR (270 MHz, CDCl₃); δ (ppm): 1.01 (9H, s), 2.65 (3H, s), 3.82 (2H, s), 8.10 (1H, s).

### Example 1-9

### 4-Amino-1-neopentyl-5-methylpyrazole (Step C2)

After degassing-nitrogen replacement of a solution of 1-neopentyl-5-methyl-4-nitropyrazole obtained in Example 1-8 in methanol (10 ml) was repeated three times, water-containing 10% palladium-carbon (palladium 4.85%) (34.05 mg, 0.1522 mmol) was added thereto. After degassing, hydrogen was replaced at 18 atm and the mixture was stirred for 3 hours. Degassing-nitrogen replacement was repeated three times and the reaction mixture was filtered using Celite. The filtrate was concentrated to obtain the desired compound (1.995 g, 11.93 mmol). Yield: 83%
Mass (EI); M/z: 167 (M⁺), 152, 110, 97, 83, 69, 56.
NMR (200 MHz, CDCl₃); δ (ppm): 0.96 (9H, s), 2.15 (3H, s), 2.63 (2H, brs), 3.74 (2H, s), 7.15 (1H, s).

### Example 2 Acetoacetic acid ester method

### Example 2-1

### Ethyl 1-isobutyl-5-methyl-4-pyrazolyl carboxylate (Step A)

Concentrated hydrochloric acid (21 ml, 252 mmol) was added dropwise to a solution of isobutylhydrazine (26 ml, 249 mmol) in ethanol (70 ml) at 0°C while stirring. A solution of ethyl 2-ethoxymethylene-3-oxobutanoate (47 g, 252 mmol) in ethanol (100 ml) was added dropwise thereto over 20 minutes and the mixture was stirred for 4 hours. The reaction mixture was concentrated up to approximately 100 ml and a saturated aqueous sodium bicarbonate solution and sodium bicarbonate were added thereto to adjust pH to 8, and the mixture was extracted with ethyl acetate. The extraction layer was dried over anhydrous magnesium sulfate and concentrated to obtain a mixture (49 g) of the desired compound and ethyl 1-isobutyl-3-methyl-4-pyrazolylcarboxylate of 10:1.
NMR (270 MHz, CDCl₃); δ (ppm): 0.91 (6H, d, J=6.4 Hz), 1.35 (3H, t, J=7.0 Hz), 2.2 (1H, brm), 2.53 (3H, s), 3.85 (2H, d, J=7.5 Hz), 4.28 (2H, q, J=7.0 Hz), 7.86 (1H, s).

### Example 2-2

### Methyl 1-isobutyl-5-methyl-4-pyrazolylcarboxylate (Step A)

Concentrated hydrochloric acid (1.0 ml, 12.0 mmol) was added dropwise to a solution of isobutylhydrazine (1.3 ml, 12.0 mmol) in methanol (10 ml) at 0°C while stirring. Methyl 2-methoxymethylene-3-oxobutanoate (1.84 g, 11.6 mmol) was added dropwise thereto and the mixture was stirred for 2 hours. A saturated aqueous sodium bicarbonate solution and sodium bicarbonate were added to the reaction mixture to adjust pH to 13 and the mixture was extracted with ethyl acetate. The extract layer was dried over anhydrous magnesium sulfate and concentrated to obtain a mixture (2 g) of the desired compound and methyl 1-isobutyl-3-methyl-4-pyrazolylcarboxylate of 17:1.
NMR (270 MHz, CDCl₃); δ (ppm): 0.91 (6H, d, J=6.4 Hz), 1.35 (3H, t, J=7.0 Hz), 2.2 (1H, brm), 2.53 (3H, s), 3.85 (2H, d, J=7.5 Hz), 4.28 (2H, q, J=7.0 Hz), 7.86 (1H, s).

### Example 2-3

### Methyl 1-allyl-5-methyl-4-pyrazolylcarboxylate (Step A)

Concentrated hydrochloric acid (2.4 ml, 22.8 mmol) was added dropwise to a solution of allylhydrazine (2.1 ml, 27.7 mmol) in methanol (15 ml) at 0°C while stirring. Methyl 2-methoxymethylene-3-oxobutanoate (4.6 g, 29.1 mmol) was added dropwise thereto and the mixture was stirred for 1 hour. The reaction mixture was added to a saturated aqueous sodium bicarbonate solution and sodium bicarbonate to adjust pH to 10 and the mixture was extracted with ethyl acetate. The extract layer was dried over anhydrous magnesium sulfate and concentrated to obtain methyl 1-allyl-3-methyl-4-pyrazolylcarboxylate (5.1 g), i.e., the desired compound.
NMR (200 MHz, CDCl₃); δ (ppm): 2.52 (3H, s), 3.82 (3H, s), 4.72 (2H, ddd, J=5.3 Hz, 1.7 Hz, 1.7 Hz), 5.0 (1H, brd, J=10.5 Hz), 5.23 (1H, brd, J=10.5 Hz), 5.86-6.03 (1H, m), 7.87 (1H, s).

### Example 2-4

### Methyl 5-methyl-1-(2-methyl-2-propenyl)-4-pyrazolylcarboxylate (Step A)

Concentrated hydrochloric acid (3.8 ml, 45.6 mmol) was added dropwise to a solution of a mixture (7 g) of methallylhydrazine and bismethallylhydrazine of approximately 5:3 in methanol (15 ml) at 0°C while stirring. Methyl 2-methoxymethylene-3-oxobutanoate (4.8 g, 30.5 mmol) was added dropwise thereto over 3 minutes and the mixture was stirred for 1 hour. The reaction mixture was added to a saturated aqueous sodium bicarbonate solution and sodium bicarbonate to adjust pH to 13 and the mixture was extracted with ethyl acetate. The extract layer was dried over anhydrous magnesium sulfate and concentrated. The thus obtained residue was purified by silica gel column chromatography (hexane~ethyl acetate:hexane = 1:5) to obtain methyl 5-methyl-1-(2-methyl-2-propenyl)-4-pyrazolylcarboxylate (2.0 g), i.e., the desired compound.
NMR (200 MHz, CDCl₃): δ (ppm): 2.51 (3H, s), 3.82 (3H, s), 4.55 (1H, brs), 4.63 (2H, brs), 4.92 (1H, brs), 7.87 (1H, s) .

### Example 2-5

### 1-Isobutyl-5-methyl-4-pyrazolylcarboxylic acid amide (Step B2, Step B4 and Step B5)

A solution of sodium hydroxide (11.6 g, 290.5 mmol) in water (35 ml) was added to a solution of methyl 1-isobutyl-5-methyl-4-pyrazolylcarboxylate (6.85 g, 34.9 mmol) in methanol (30 ml) at room temperature and the mixture was stirred for 2.5 hours. After the mixture was cooled to 0°C, concentrated hydrochloric acid was added thereto to adjust pH to 1. After the mixture was extracted with ethyl acetate, it was dried over anhydrous magnesium sulfate and concentrated to obtain 1-isobutyl-5-methyl-4-pyrazolylcarboxylic acid as pale yellow crystals.

Thionyl chloride (3.2 ml, 43.87 mmol) was added dropwise to a solution of the obtained crystals in dichloromethane (20 ml) at 0°C and thereafter the mixture was refluxed for 1.5 hours. The mixture was cooled to room temperature and concentrated to obtain 1-isobutyl-5-methyl-4-pyrazolylcarbonyl chloride as an oil.

25% Aqueous ammonia (8 ml, 106 mmol) was added dropwise to a solution of the obtained oil in tetrahydrofuran (20 ml) at 0°C and the mixture was stirred for 20 minutes. Water was added to the reaction mixture and the mixture was extracted with ethyl acetate. The extract layer was dried over anhydrous magnesium sulfate and concentrated. The thus obtained residue was purified by silica gel column chromatography (hexane-ethyl acetatemethanol:ethyl acetate = 1:20) to obtain the desired 1-isobutyl-5-methyl-4-pyrazolylcarboxylic acid amide as a pale yellow powder (4.65 g, 25.66 mmol).
NMR (270 MHz, CDCl₃); δ (ppm): 0.92 (6H, d, J=7.0 Hz), 2.22 (1H, m), 2.56 (3H, s), 3.86 (2H, d, J=7.5 Hz), 5.5 (2H, brs), 7.64 (1H, s).

### Example 2-6

### 4-Amino-1-isobutyl-5-methylpyrazole (Step B6)

Approximately 21% aqueous sodium hypochlorite solution (7.2 ml, 24 mmol) was added dropwise to an aqueous sodium hydroxide (1.78 g, 44.42 mmol) solution (10 ml) at 0°C and the mixture was stirred at 10°C for 5 minutes. 1-Isobutyl-5-methyl-4-pyrazolylcarboxylic acid amide (730.2 mg, 4.03 mmol) was added thereto and the mixture was stirred at 50°C for 2 hours and at 70°C for 1 hour. After the mixture was cooled to 0°C, concentrated hydrochloric acid was added thereto until pH became 1.0 and the mixture was stirred at room temperature for 30 minutes. Sodium hydroxide was added thereto to adjust pH to 14 and the mixture was extracted with ethyl acetate. The extracts were dried and concentrated to obtain the desired product (505.8 mg).
NMR (200 MHz, CDCl₃); δ (ppm): 0.89 (6H, d, J=7.0 Hz), 2.15 (3H, s), 2.15 (1H, brquint, J=7.0 Hz), 2.49 (2H, brs), 2.75 (1H, brquint, J=7.6 Hz), 3.96 (2H, d, J=7.6 Hz), 7.12 (1H, s).

### Example 2-7

### Ethyl 1-isobutyl-5-methyl-4-pyrazolylcarboxylate (Step C)

Degassing and nitrogen-replacement of a solution of methyl 5-methyl-1-(2-methyl-2-propenyl)-4-pyrazolylcarboxylate (196.9 mg, 1.014 mmol) in methanol (10 ml) were carried out three times. Water-containing 7.5% palladium-carbon catalyst (116 mg, 0.05 mmol) was added thereto, and after degassing, hydrogen-replacement was carried out and the mixture was stirred for 1 hour. After degassing and nitrogen-replacement were carried out three times, the mixture was filtered using Celite and the filtrate was concentrated to obtain the desired compound (186.2 mg).

### Example 3 Acetylacetone method

### Example 3-1

### 1-(1-Isobutyl-5-methyl-1H-pyrazol-4-yl)ethanone

### (Step A)

Concentrated hydrochloric acid (17.5 ml, 210 mmol) was added to a solution of isobutylhydrazine (18.5 g, 210 mmol) in ethanol (100 ml) at 0°C and the mixture was stirred for 10 minutes. A solution of 3-ethoxymethylenepentane-2,4-dione (31.24 g, 200 mmol) in ethanol (200 ml) was added dropwise thereto at the same temperature over 40 minutes and the mixture was stirred at room temperature for 1.5 hours. After completion of the reaction, ethanol was evaporated under reduced pressure. A saturated aqueous sodium bicarbonate solution was poured to the obtained residue to neutralize it and the mixture was extracted with ethyl acetate. The extract layer was washed with brine once and dried over anhydrous sodium sulfate. The solution was concentrated and the obtained residue was purified by silica gel column chromatography (hexane:ethyl acetate = 6:4) to obtain a mixture (35.25 g, 196 mmol; HPLC ratio 98.9:1.1) of the desired product and 1-(1-isobutyl-3-methyl-1H-pyrazole-4-yl)ethanone. Yield: 98%
Mass (EI); m/z: 180 (M⁺), 165, 137, 125, 109, 95.
NMR (200 MHz, CDCl₃); δ (ppm): 0.92 (6H, d, J=7.0 Hz), 2.23 (1H, hep, J=7.0 Hz), 2.43 (3H, s), 2.56 (3H, s), 3.86 (2H, d, J=7.0 Hz), 7.84 (1H, s).

### Example 3-2

### 1-(1-Isobutyl-5-methyl-1H-pyrazol-4-yl)ethanone oxime

### (Step B1)

Hydroxylamine hydrochloride (4.90 g, 68.4 mmol) and pyridine (5.5 ml, 68.4 mmol) were successively added to a solution of 1-(1-isobutyl-5-methyl-1H-pyrazol-4-yl)ethanone (10.28 g, 57.0 mmol) in methanol (30 ml) at room temperature and the mixture was stirred for 8 hours. After completion of the reaction, methanol was evaporated under reduced pressure and the residue was diluted with ethyl acetate. The organic layer was washed with brine twice and dried over anhydrous sodium sulfate. The solution was concentrated and the obtained crude crystals was washed with hexane and dried to obtain the desired product (9.92 g, 50.8 mmol). Yield: 89%
Mass (EI); m/z: 195 (M⁺), 180, 152, 139, 122, 107.
NMR (200 MHz, CDCl₃); δ (ppm): 0.91 (6H, d, J=7.0 Hz), 2.18 (1H, hep, J=7.0 Hz), 2.21 (3H, s), 2.43 (3H, s), 3.86 (2H, d, J=7.0 Hz), 7.57 (1H, s), 7.79 (1H, brs).

### Example 3-3

### 1-Isobutyl-5-methyl-1H-pyrazol-4-amine (Step B2)

Concentrated sulfuric acid (27 ml, 507 mmol) was added to 1-(1-isobutyl-5-methyl-1H-pyrazol-4-yl)ethanone oxime (9.92 g, 50.8 mmol) at room temperature and the mixture was stirred at 160°C for 90 minutes. After being air-cooled to room temperature, the reaction mixture was poured to distilled water (55 ml) and the mixture was heated under reflux for 30 minutes. After completion of the reaction, 25% aqueous ammonia (70 ml) was poured thereto under ice-cooling to make it alkaline and the mixture was extracted with ethyl acetate twice. The organic layer was washed with brine once and dried over anhydrous sodium sulfate. The solution was concentrated to obtain 1-isobutyl-5-methyl-1H-pyrazol-4-amine (6.55 g, 42.7 mmol). Yield: 84%
Mass (EI); m/z: 153 (M⁺), 138, 110.
NMR (200 MHz, CDCl₃); δ (ppm): 0.89 (6H, d, J=7.0 Hz), 2.15 (3H, s), 2.16 (1H, hep, J=7.0 Hz), 2.65 (2H, brs), 3.75 (2H, d, J=7.0 Hz), 7.15 (1H, s).

### Example 3-4

### 1-{5-methyl-1-(2-methyl-2-propenyl)-1H-pyrazol-4-yl}ethanone (Step A)

Concentrated hydrochloric acid (2.7 ml, 32.4 mmol) was added to a solution of methalylhydrazine (0.91 g, 10.6 mmol) in methanol (5 ml) at 0°C and the mixture was stirred for 30 minutes. A solution of 3-methoxymethylenepentane-2,4-dione (1.43 g, 10.1 mmol) in methanol (5 ml) was added dropwise thereto at the same temperature and the mixture was stirred at 0°C for 2 hours. After completion of the reaction, a saturated aqueous sodium bicarbonate solution was poured to the reaction solution to neutralize it and the mixture was extracted with ethyl acetate. The extract layer was washed with brine once and dried over anhydrous sodium sulfate. The solution was concentrated to obtain a mixture (1.74 g, 9.8 mmol; HPLC ratio 98.2:1.8) of the desired product and 1-(3-methyl)-1-(2-methyl-2-propenyl)-1H-pyrazol-4-yl)ethanone. Yield: 97%
NMR (270 MHz, CDCl₃); δ (ppm): 1.70 (3H, s), 2.47 (3H, s), 2.53 (3H, s), 4.51 (1H, brs), 4.55 (2H, brs), 4.94 (1H, brs), 7.85 (1H, s).

### Example 3-5

### 1-(1-Isobutyl-5-methyl-1H-pyrazol-4-yl)ethanone

### (Step B1)

A solution of 1-{5-methyl-1-(2-methyl-2-propenyl)-1H-pyrazol-4-yl}ethanone (1.39 g, 7.80 mmol) in methanol (15 ml) was added to 10% water-containing palladium-carbon catalyst (0.09 g) under a nitrogen atmosphere. After degassing, hydrogen replacement is carried out and the mixture was stirred at room temperature for 2 hours. After degassing and hydrogen-replacement, the palladium catalyst was separated by filtration using Celite and the filtrate was concentrated to obtain the desired product (1.39 g). Yield: 99%

### Effect of invention

By the present invention, the 4-amino-5-methylpyrazole derivatives being useful as synthetic intermediates of pharmaceuticals, agricultural chemicals or the like can be efficiently synthesized.

## Claims

1. A 4-amino-5-methylpyrazole derivative represented by the following formula (I): 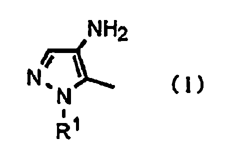 (wherein R¹ represents a group selected from group a, group b or group c <<provided that a methyl group is excluded>>, the group a is the group comprising a C₁-C₆ alkyl group which is the straight or branched alkyl group and may have optionally a C₃-C₆ alkyl group(s) as a substituent(s), the group b is the group comprising a straight or branched alkyl group which may have optionally a substituent(s), a straight or branched alkenyl group which is the alkenyl group bonded at the sp³ carbon atom and which may have optionally a substituent(s), a straight or branched alkynyl group which is the alkynyl group bonded at the sp³ carbon atom and which may have optionally a substituent(s) or a cycloalkenylmethyl group, and the group c is the group comprising a straight or branched alkyl group having 3 or more carbon chains which may have optionally a substituent(s), a cycloalkylmethyl group which may have optionally a substituent(s), a straight or branched alkenyl group which is the alkenyl group bonded at the sp³ carbon atom and which may have optionally a substituent(s), a straight or branched alkynyl group which is the alkynyl group bonded at the sp³ carbon atom and which may have optionally a substituent(s) or a cycloalkenylmethyl group)
or a salt thereof.

2. A process for preparing a 4-amino-5-methylpyrazole derivative represented by the following formula (Ia): 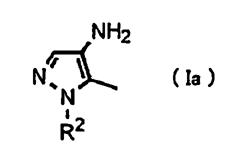 (wherein R² represents a C₁-C₆ alkyl group which is a straight or branched alkyl group and may have optionally a C₃-C₆ cycloalkyl group(s) as a substituent(s)),
comprising a step for obtaining a compound represented by the following formula (1-1): 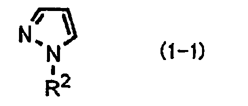 (wherein R² has the same meaning as defined above) by reacting 1H-pyrazole with a halogenated alkyl in two layer system of an aqueous sodium hydroxide solution and an organic solvent using a phase transfer catalyst.

3. A process for preparing a 4-amino-5-methylpyrazole derivative represented by the following formula (Ia): 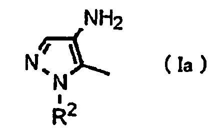 (wherein R² represents a C₁-C₆ alkyl group which is the straight or branched alkyl group and may have optionally a C₃-C₆ cycloalkyl group(s) as a substituent(s)),
comprising a step for obtaining a compound represented by the following formula (1-2): 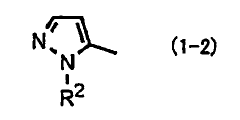 (wherein R² has the same meaning as defined above) by lithiating selectively the 5-position of the pyrazole of a compound represented by the following formula (1-1): 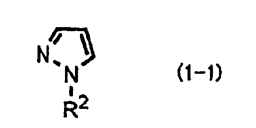 (wherein R² has the same meaning as defined above) in tetrahydrofuran using an alkyl lithium and reacting it with a methylating agent.

4. A process for preparing a 4-amino-5-methylpyrazole derivative represented by the following formula (Ia): 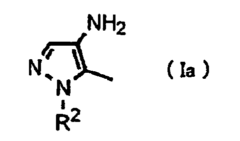 (wherein R² represents a C₁-C₆ alkyl group which is a straight or branched alkyl group and may have optionally a C₃-C₆ cycloalkyl group(s) as a substituent(s)),
comprising nitrating a compound represented by the following formula (1-2): 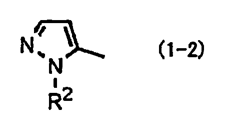 (wherein R² has the same meaning as defined above) and reducing it.

5. A compound represented by the following formula (1-3): 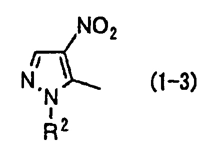 (wherein R² represents a C₁-C₆ alkyl group which is the straight or branched alkyl group and may have optionally a C₃-C₆ cycloalkyl group(s) as a substituent(s)).

6. A compound represented by the following formula (2-1): 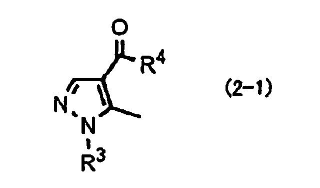 (wherein R³ represents a straight or branched alkenyl group which is the alkenyl group bonded at the sp³ carbon atom and may have optionally a substituent(s), a straight or branched alkynyl group which is the alkynyl group bonded at the sp³ carbon atom and may have optionally a substituent(s) or a cycloalkenylmethyl group; and R⁴ represents a hydroxyl group, a lower alkoxy group or an amino group).

7. A process for preparing a compound represented by the following formula (2-4): 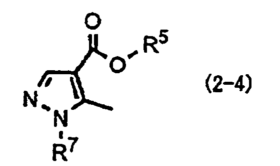 (wherein R⁵ represents a lower alkyl group and R⁷ represents a straight or branched alkyl group which may have optionally a substituent(s), a straight or branched alkenyl group which is the alkenyl group bonded at the sp³ carbon atom and may have optionally a substituent(s), a straight or branched alkynyl group which is the alkynyl group bonded at the sp³ carbon atom and may have optionally a substituent(s) or a cycloalkenylmethyl group),
comprising reacting a compound represented by the following formula (2-2): 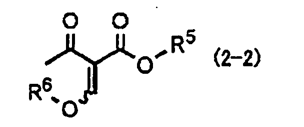 (wherein R⁵ has the same meaning as defined above and R⁶ independently represents a lower alkyl group) with a compound represented by the following formula (2-3) : 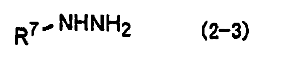 (wherein R⁷ has the same meaning as defined above) under co-existence of an acid.

8. A compound represented by the following formula (2-4): 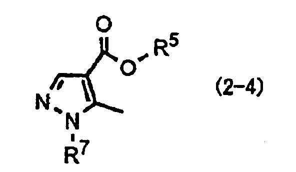 (wherein R⁵ represents a lower alkyl group and R⁷ represents a straight or branched alkyl group which may have optionally a substituent(s), a straight or branched alkenyl group which is the alkenyl group bonded at the sp³ carbon atom and may have optionally a substituent(s), a straight or branched alkynyl group which is the alkynyl group bonded at the sp³ carbon atom and may have optionally a substituent(s) or a cycloalkenylmethyl group).

9. A process for preparing a compound represented by the following formula (2-5): 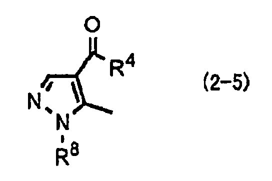 (wherein R⁴ represents a hydroxyl group, a lower alkoxy group or an amino group; and R⁸ represents a straight or branched alkyl group which may have optionally a substituent(s)),
comprising hydrogenating a compound represented by the following formula (2-1): 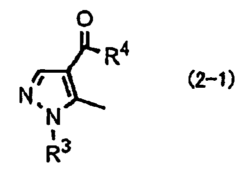 (wherein R³ represents a straight or branched alkenyl group which is the alkenyl group bonded at the sp³ carbon atom and may have optionally a substituent(s), a straight or branched alkynyl group which is the alkynyl group bonded at the sp³ carbon atom and may have optionally a substituent(s) or a cycloalkenylmethyl group; and R⁴ has the same meaning as defined above).

10. A compound represented by the following formula (2-5): 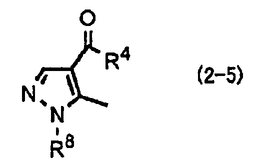 (wherein R⁴ represents a hydroxyl group, a lower alkoxy group or an amino group; and R⁸ represents a straight or branched alkyl group which may have optionally a substituent(s)).

11. A process for preparing a 4-amino-5-methylpyrazole derivative represented by the formula (Ib), comprising obtaining the 4-amino-5-methylpyrazole derivative represented by the following formula (Ib): 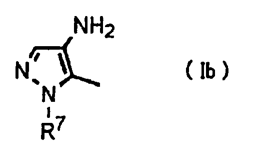 (wherein R⁷ represents a straight or branched alkyl group which may have optionally a substituent(s), a straight or branched alkenyl group which is the alkenyl group bonded at the sp³ carbon atom and may have optionally a substituent(s), a straight or branched alkynyl group which is the alkynyl group bonded at the sp³ carbon atom and may have optionally a substituent(s) or a cycloalkenylmethyl group)
by converting the functional group of a compound represented by the following formula (2-4): 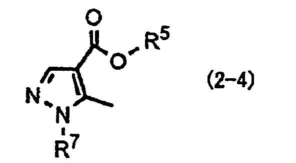 (wherein R⁵ represents a lower alkyl group; and R⁷ has the same meaning as defined above)
and by carrying out Hofmann rearrangement reaction, Schmidt rearrangement reaction, Curtius rearrangement reaction or Lossen rearrangement reaction.

12. A compound represented by the following formula (3-1): 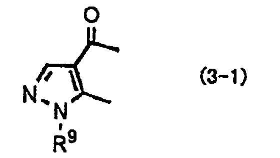 (wherein R⁹ represents a straight or branched alkyl group having 3 or more carbon chains which may have optionally a substituent(s), a cycloalkylmethyl group which may have optionally a substituent(s), a straight or branched alkenyl group which is the alkenyl group bonded at the sp³ carbon atom and may have optionally a substituent(s), a straight or branched alkynyl group which is the alkynyl group bonded at the sp³ carbon atom and may have optionally a substituent(s) or a cycloalkenylmethyl group).

13. A process for preparing a compound represented by the following formula (3-1): 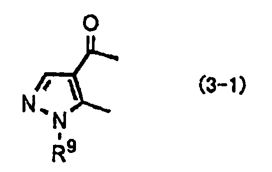 (wherein R⁹ represents a straight or branched alkyl group having 3 or more carbon chains which may have optionally a substituent(s), a cycloalkylmethyl group which may have optionally a substituent(s), a straight or branched alkenyl group which is the alkenyl group bonded at the sp³ carbon atom and may have optionally a substituent(s), a straight or branched alkynyl group which is the alkynyl group bonded at the sp³ carbon atom and may have optionally a substituent(s) or a cycloalkenylmethyl group),
comprising reacting a compound represented by the following formula (3-2): 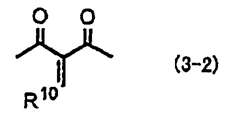 (wherein R¹⁰ represents a lower alkoxy group, a phenoxy group or a dialkylamino group)
with a compound represented by the following formula (3-3) : 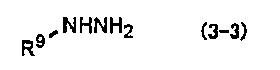 (wherein R⁹ has the same meaning as defined above) under co-existence of an acid.

14. A process for preparing a compound represented by the following formula (3-1"): 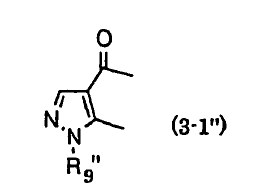 (wherein R₉" represents a straight or branched alkyl group having 3 or more carbon chains which may have optionally a substituent(s) or a cycloalkylmethyl group which may have optionally a substituent(s)),
comprising hydrogenating a compound represented by the following formula (3-1'): 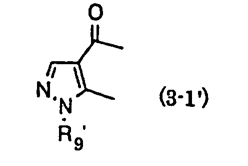 (wherein R₉' represents a straight or branched alkenyl group which is the alkenyl group bonded at the sp³ carbon atom and may have optionally a substituent(s), a straight or branched alkynyl group which is the alkynyl group bonded at the sp³ carbon atom and may have optionally a substituent(s) or a cycloalkenylmethyl group).

15. A compound represented by the following formula (3-4): 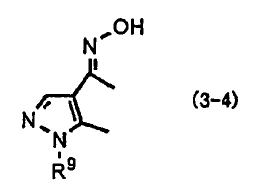 (wherein R₉ represents a straight or branched alkyl group having 3 or more carbon chains which may have optionally a substituent(s), a cycloalkylmethyl group which may have optionally a substituent(s), a straight or branched alkenyl group which is the alkenyl group bonded at the sp³ carbon atom and may have optionally a substituent(s), a straight or branched alkynyl group which is the alkynyl group bonded at the sp³ carbon atom and may have optionally a substituent(s) or a cycloalkenylmethyl group).

16. A process for preparing a 4-amino-5-methylpyrazole derivative represented by the formula (Ic), comprising obtaining the 4-amino-5-methylpyrazole derivative represented by the following formula (Ic): 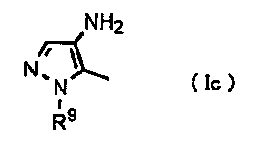 (wherein R₉ represents a straight or branched alkyl group having 3 or more carbon chains which may have optionally a substituent(s), a cycloalkylmethyl group which may have optionally a substituent(s), a straight or branched alkenyl group which is the alkenyl group bonded at the sp³ carbon atom and may have optionally a substituent(s), a straight or branched alkynyl group which is the alkynyl group bonded at the sp³ carbon atom and may have optionally a substituent(s) or a cycloalkenylmethyl group) by carrying out Beckman rearrangement reaction from a compound represented by the following formula (3-4): 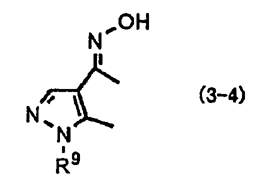 (wherein R₉ has the same meaning as defined above).
